# FASCICULE DE BREVET EUROPEEN

(11) **EP 2 977 385 B1**
(45) Date de publication et mention de la délivrance du brevet: **27.12.2017**
(21) Numéro de dépôt: 15160538.3
(22) Date de dépôt: 11.05.2010
(51) Int. Cl.: C07K 16/18, G01N 33/68, A61K 39/395, A61P 25/28

(54) **ANTICORPS HUMANISES SPÉCIFIQUES DE LA FORME PROTOFIBRILLAIRE DU PEPTIDE BÉTA-AMYLOIDE**
SPEZIFISCHE HUMANISIERTE ANTIKÖRPER DER PROTOFIBRILLÄREN FORM DES BETA-AMYLOID-PEPTIDS
HUMANISED ANTIBODIES SPECIFIC TO THE PROTOFIBRILLAR FORM OF THE AMYLOID-BETA PEPTIDE

(30) Priorité: 12.05.2009 FR 0953133
(43) Date de publication de la demande: 27.01.2016
(62) Demande divisionnaire de: 10728743.5
(73) Titulaire: SANOFI, 75008 Paris (FR)
(72) Inventeur: Baurin, Nicolas, 75008 Paris (FR); Blanche, Francis, 75013 Paris (FR); Cameron, Béatrice, 75008 Paris (FR); Duchesne, Marc, 75013 Paris (FR); Mikol, Vincent, 75008 Paris (FR); Naimi, Souad, 75008 Paris (FR); Pradier, Laurent, 75013 Paris (FR); Shi, Yi, 75008 Paris (FR)
(74) Mandataire: Lavoix

(56) Documents cités:
- WO-A1-2005/123775
- WO-A1-2006/081171
- WO-A1-2007/108756
- WO-A1-2008/081008
- WO-A2-02/46237
- WO-A2-2006/036291
- WO-A2-2009/065054
- SCHUPF NICOLE ET AL: "Peripheral Abeta subspecies as risk biomarkers of Alzheimer's disease.", PROCEEDINGS OF THE NATIONAL ACADEMY OF SCIENCES OF THE UNITED STATES OF AMERICA 16 SEP 2008, vol. 105, no. 37, 16 septembre 2008 (2008-09-16), pages 14052-14057, XP002567834, ISSN: 1091-6490
- R. ROBERT ET AL: "Engineered antibody intervention strategies for Alzheimer's disease and related dementias by targeting amyloid and toxic oligomers", PROTEIN ENGINEERING DESIGN AND SELECTION, vol. 22, no. 3, 16 octobre 2008 (2008-10-16), pages 199-208, XP055070400, ISSN: 1741-0126, DOI: 10.1093/protein/gzn052

## Description

La présente invention a pour objet des anticorps humanisés spécifiques de la forme protofibrillaire du peptide β-amyloïde. La présente invention a également pour objet l'utilisation thérapeutique, diagnostique et/ou préventive de ces anticorps, en particulier associée au déclenchement et à la progression de troubles neurodégénératifs et/ou aux maladies liées au dépôt de plaques amyloïdes, et notamment de la maladie d'Alzheimer.

La maladie d'Alzheimer (AD) est une maladie neurodégénérative progressive qui affecte une large proportion de la population âgée. Cette maladie est caractérisée sur le plan clinique par une perte de la mémoire et un déclin des fonctions cognitives, sur le plan neuropathologique par la présence dans le cerveau de dépôts neurofibrillaires intracellulaires et de dépôts extracellulaires du peptide β-amyloïde (A-β) formant les plaques amyloïdes. (Yanker et al. Nature Med. Vol 2 No 8 (1996)). A ces signes s'ajoutent un nombre important d'autres changements anormaux incluant une altération des systèmes immunitaires et inflammatoires ainsi qu'une altération de la fonction mitochondriale pouvant conduire à une augmentation du stress oxydatif, une activation des mécanismes de l'apoptose et de manière ultime une mort cellulaire.

Les plaques amyloïdes sont majoritairement composées des peptides A-β à 40 ou 42 résidus qui sont générés lors du processus protéolytique de la protéine précurseur du peptide β-amyloïde (APP). Les dépôts extracellulaires de peptides A-β représentent la caractéristique précoce et invariable de toutes les formes de l'AD, incluant les formes familiales (FAD). Les FAD apparaissent de manière relativement précoce (entre 40 et 60 ans) et sont dues à des mutations dans le gène de l'APP dans 5 % des cas de FAD (> 20 familles) avec six mutations faux-sens simples ou doubles; dans le gène de la préséniline 1 (PS 1) dans 50 à 70 % des cas de FAD (> à 200 familles) avec plus de 80 mutations différentes identifiées jusqu'à présent; et dans le gène de la préséniline 2 (PS 2) dans moins de cas de FAD avec 2 mutations faux-sens décrites dans 8 familles. Des mutations dans ces trois gènes ont été démontrées comme induisant des changements dans la protéolyse de l'APP, qui conduisent à une surproduction de A-β et à l'apparition précoce de la pathologie et des symptômes qui sont similaires à ceux des formes sporadiques de l'AD.

La toxicité neuronale des plaques amyloïdes pourrait résider dans les fibrilles de poids moléculaire important qui sont formés par agrégation de peptides A-β solubles en formes fibrillaires d'abord solubles (dite aussi forme protofibrillaire) qui sont ensuite converties en formes insolubles incorporées dans les plaques amyloïdes. En effet in vitro il a été montré que le peptide A-β soluble s'agrégeait progressivement en une forme fibrillaire (c-a-d qui peut être marqué par les agents tels que le rouge congo ou la thioflavine S qui reconnaissent les structures tertiaires en feuillet beta des peptides/protéines), de haut poids moléculaire (>200 kDa) mais encore soluble. Parce que cette forme est soluble, elle est souvent appelée forme protofibrillaire tandis que les fibrilles résultent d'une agrégation encore plus importante conduisant à la perte de solubilité. Les formes protofibrillaires de transition sont généralement considérées comme les précurseurs des fibres amyloïdes et pourraient être responsables du dysfonctionnement cellulaire et de la perte neuronale dans la maladie d'Alzheimer et dans d'autres maladies liées à l'agrégation de protéines.

Il a été montré que les plaques amyloïdes séniles (c'est-à-dire agrégées, on les appelle aussi plaques matures) sont corrélées avec le statut cognitif des patients Alzheimer contrairement aux dépôts diffus de peptide A-β qui sont aussi largement présents chez les patients non-atteints. (Duyckaerts et Al, Neurobiol.Aging 1997 ;18 : 33-42 et et Jellinger et Al. 1998; 54 :77-95). Cibler en particulier ces plaques amyloïdes séniles permet donc de traiter la pathologie Alzheimer de manière plus spécifique et efficace.

De nombreux traitements ont été tentés afin de prévenir la formation des peptides A-β, comme par exemple des inhibiteurs du processus protéolytique de l'APP.
Des stratégies d'immunothérapie comme l'administration d'anticorps anti-A-β (pour diminuer les dépôts amyloïdes) ou l'immunisation avec des antigènes des peptides A-β (pour promouvoir une réponse humorale) ont été testées afin de réduire la taille et la densité des plaques.
On a par exemple décrit (US 7 179 463) une méthode de traitement contre la maladie d'Alzheimer consistant à administrer un anticorps dirigé contre un protofibrille présentant une mutation Arctic à l'intérieur de la région codant pour le peptide A-β. Aucun exemple d'anticorps n'est réellement décrit. De plus, aucune comparaison de l'affinité des anticorps pour les peptides en fonction du poids moléculaire de ces peptides n'a été réalisée. D'autres brevets (US 6 761 888 et US 6 750 324) ont évoqué des anticorps reconnaissant diverses épitopes le long de la séquence en acides aminés du peptide A-β₄₂. Une demande internationale (WO2007/108756) a été déposée concernant des anticorps spécifiques des protofibrilles mais les anticorps décrits reconnaissent à la fois les peptides A-β de haut poids moléculaire et les oligomères de poids moyen. De plus l'affinité des anticorps pour les plaques matures par rapport à leur affinité pour les plaques diffuses n'est pas évoquée.
Schupf et al. (PNAS, 2008, vol. 105, n° 37, p 14052-14057) décrit la génération d'un anticorps murin (13C3) par immunisation avec la forme fibrillaire de la protéine Aβ42. L'anticorps 13C3 est spécifique de la forme protofibrillaire du peptide Aβ42 et ne réagit pas avec les fractions de faible poids moléculaire.

Malgré l'évolution actuelle des connaissances à propos de la maladie d'Alzheimer, il existe toujours un besoin pour des compositions et des méthodes de traitement et/ou de prévention de cette pathologie limitant au maximum les effets secondaires. Des anticorps tels que décrits dans la présente demande, humanisés et spécifiques de la forme protofibrillaire des peptides A-β ont pour but de résoudre ce problème. Permettant la reconnaissance des plaques amyloïdes séniles et non des plaques diffuses, les anticorps objet de l'invention reconnaissent les plaques pathologiques de façon beaucoup plus efficace que des anticorps reconnaissant toutes les formes d'Abeta, qui seront en grande partie fixés sur les dépôts diffus ou fixée sur les formes solubles de peptide A-β monomérique ou de bas poids moléculaire.

De plus, le fait de reconnaître uniquement les formes protofibrillaires des peptides A-β et non les formes protofibrillaires d'autres protéines non liées à la maladie d'Alzheimer évite des liaisons inutiles susceptibles de diminuer la concentration d'anticorps efficaces sur la maladie.

L'anticorps murin que l'on a humanisé portera le nom d'anticorps 13C3 dans toute la présente demande.
Les séquences pouvant coder pour ou constituer les anticorps humanisés décrits dans la présente demande figurent dans le Tableau 2.
La présente invention est définie par les revendications.
La présente demande décrit un anticorps humanisé se liant spécifiquement à la forme protofibrillaire du peptide A-β, soit un peptide de haut poids moléculaire.
Dans un mode plus avantageux, l'anticorps se lie au peptide A-β ayant un poids moléculaire supérieur à 200, 300, 400 ou 500 kDa.
Il est également décrit que l'anticorps se lie aux peptides A-β agrégés en plaques séniles et non aux dépôts diffus de peptides A-β.

Dans un mode de réalisation avantageux, l'anticorps décrit ici se lie spécifiquement aux formes protofibrillaires du peptide A-β mais pas aux autres protéines de structure amyloïdes (comme par exemple les IAPP Islet Amyloid Polypeptide).
La présente demande décrit également un anticorps humanisé ayant des fonctions effectrices diminuées, ce qui permet une limitation des effets adverses tels que l'apparition de micro-hémorragies et oedèmes vasogéniques.
Dans un mode de réalisation avantageux, l'anticorps ne possède plus de fonctions effectrices.
Dans un mode de réalisation encore plus avantageux, l'anticorps est une immunoglobuline G 4 dont le domaine Fc a subi des mutations diminuant la production de demi-molécules.
Dans un mode de réalisation encore plus avantageux, l'anticorps est une immunoglobuline G 4 dont le domaine Fc a subi des mutations diminuant l'activité effectrice.

La présente demande décrit un anticorps humanisé comprenant au moins un CDR codé par une séquence nucléotidique présentant une séquence identique à l'une des séquences SEQ ID NO : 9, 11, 13, 15, 17 et 19, ou par des séquences différant respectivement par 1, 2, 3, 4 ou 5 nucléotides de ces séquences.
La présente demande décrit également un anticorps humanisé comprenant au moins un CDR présentant une séquence identique à l'une des séquences SEQ ID NO : 10, 12, 14, 16, 18 et 20.

Dans un autre mode de réalisation, l'anticorps décrit ici comprend au moins un CDR dont la séquence diffère de un à deux acides aminés par rapport à l'une des séquences SEQ ID NO : 10, 12, 14, 16, 18, 20 et 32, pour autant que l'anticorps garde sa spécificité de liaison.

Dans un mode de réalisation avantageux, l'anticorps comprend les CDR codés par les séquences nucléotidiques SEQ ID NO: 9, 11, 13, 15, 17 et 19, ou par des séquences différant respectivement par 1,2,3,4 ou 5 nucléotides de ces séquences. Dans un autre mode de réalisation avantageux, l'anticorps comprend les CDR de séquence SEQ ID NO : 10, 12, 14, 16, 18 et 20.

L'anticorps peut aussi comprendre les CDR codés par les séquences nucléotidiques SEQ ID NO : 9, 11, 13, 31, 17 et 19 ou par des séquences différant respectivement par 1, 2, 3, 4 ou 5 nucléotides de ces séquences.
Dans un mode de réalisation avantageux, l'anticorps comprend les CDR de séquence SEQ ID NO : 10, 12, 14, 32, 18 et 20.

La présente demande décrit l'anticorps humanisé comprenant les CDR codés par les séquences nucléotidiques SEQ ID NO : 9, 11, 29, 31, 17 et 19 ou par des séquences différant respectivement par 1, 2, 3, 4 ou 5 nucléotides de ces séquences.
Est également décrit un anticorps humanisé comprenant les CDR de séquence SEQ ID NO : 10, 12, 30, 32, 18 et 20.

Dans un mode de réalisation avantageux, l'anticorps décrit ici comprend une partie variable de sa chaîne lourde (VH) codée par une séquence présentant au moins 80%, 85%, 90%, 95% ou 99% d'identité avec la séquence SEQ ID NO : 5 ou la séquence SEQ ID NO 27.
Dans un mode de réalisation avantageux, l'anticorps décrit ici comprend une partie variable de sa chaîne lourde (VH) comprenant une séquence présentant au moins 80%, 85%, 90%, 95% ou 99% d'identité avec la séquence SEQ ID NO : 6 ou la séquence SEQ ID NO 28.
Dans un mode de réalisation avantageux, l'anticorps décrit ici comprend une partie variable de sa chaîne légère (VL) codée par une séquence présentant au moins 80%, 85%, 90%, 95% ou 99% d'identité avec la séquence SEQ ID NO : 7 ou la séquence SEQ ID NO 23.
Dans un mode de réalisation avantageux, l'anticorps décrit ici comprend une partie variable de sa chaîne légère (VL) comprenant une séquence présentant au moins 80%, 85%, 90%, 95% ou 99% d'identité avec la séquence SEQ ID NO : 8 ou la séquence SEQ ID NO 24.

Dans un mode de réalisation encore plus avantageux, l'anticorps comprend une chaîne lourde comprenant une partie variable (VH) codée par l'une des séquences nucléotidiques SEQ ID NO 5 et SEQ ID NO 27.

Dans un mode de réalisation encore plus avantageux, l'anticorps comprend une chaîne lourde comprenant une partie variable (VH) de séquence polypeptidique SEQ ID NO 6 ou SEQ ID NO 28.

Dans un autre mode de réalisation, l'anticorps comprend une chaîne légère comprenant une partie variable (VL) codée par l'une des séquences nucléotidiques SEQ ID NO 7 et SEQ ID NO 23.
Dans un mode autre de réalisation, l'anticorps comprend une chaîne légère comprenant une partie variable (VL) de séquence polypeptidique SEQ ID NO 8 ou SEQ ID NO 24.

Dans un mode de réalisation avantageux, l'anticorps comprend les séquences codées par les séquences nucléotidiques SEQ ID NO : 5 et 7.
Dans un mode de réalisation avantageux, l'anticorps comprend les séquences polypeptidiques SEQ ID NO : 6 et 8.

Dans un autre mode de réalisation, l'anticorps comprend les séquences codées par les séquences nucléotidiques SEQ ID NO : 5 et 23.
Dans un autre mode de réalisation, l'anticorps comprend les séquences polypeptidiques SEQ ID NO : 6 et 24.

Dans un autre mode de réalisation, l'anticorps comprend les séquences codées par les séquences nucléotidiques SEQ ID NO : 27 et 23.
Dans un autre mode de réalisation, l'anticorps comprend les séquences polypeptidiques SEQ ID NO : 28 et 24.

La présente demande décrit également un anticorps comprenant une chaîne lourde codée par une séquence présentant au moins 80%, 85%, 90%, 95% ou 99% d'identité avec l'une des séquences nucléotidiques SEQ ID NO 1 et SEQ ID NO 25.

La présente demande décrit également un anticorps comprenant une chaîne lourde présentant au moins 80%, 85%, 90%, 95% ou 99% d'identité avec la séquence polypeptidique SEQ ID NO 2 ou avec la séquence polypeptidique SEQ ID NO 26.

Dans un mode de réalisation avantageux l'anticorps décrit ici comprend une chaîne légère codée par une séquence présentant au moins 80%, 85%, 90%, 95% ou 99% d'identité avec l'une des séquences nucléotidiques SEQ ID NO 3 et SEQ ID NO 21. Dans un autre mode de réalisation l'anticorps décrit ici comprend une chaîne légère comprenant une séquence présentant au moins 80%, 85%, 90%, 95% ou 99% d'identité avec l'une des séquences polypeptidiques SEQ ID NO 4 et SEQ ID NO 22.

La présente demande décrit un anticorps comprenant les séquences codées par les séquences nucléotidiques SEQ ID NO : 1 et 3.
La présente demande décrit un anticorps dont la séquence comprend les séquences polypeptidiques SEQ ID NO : 2 et 4.

La présente demande décrit un anticorps comprenant les séquences codées par les séquences nucléotidiques SEQ ID NO : 1 et 21.
La présente demande décrit un anticorps dont la séquence comprend les séquences polypeptidiques SEQ ID NO : 2 et 22.

La présente demande décrit un anticorps comprenant les séquences codées par les séquences nucléotidiques SEQ ID NO : 25 et 21.
La présente demande décrit un anticorps dont la séquence comprend les séquences polypeptidiques SEQ ID NO : 26 et 22.

La présente demande décrit un anticorps humanisé anti-peptide Aβ ayant une affinité pour la forme protofibrillaire du peptide Aβ au moins 100 fois supérieure à son affinité pour les autres formes de ce peptide.

La présente demande décrit un anticorps caractérisé en ce qu'il induit une diminution des plaques amyloïdes.

La présente demande décrit l'utilisation d'un anticorps humanisé anti-peptide Aβ dans le traitement des maladies associées aux troubles neuro-dégénératifs, et en particulier dans le traitement de la maladie d'Alzheimer.
La présente demande décrit une composition pharmaceutique comprenant un anticorps humanisé anti-peptide Aβ et des excipients.

La présente demande décrit une méthode de traitement de la maladie d'Alzheimer comprenant l'administration au patient d'un anticorps humanisé anti peptide- Aβ.

La présente demande décrit une cellule ou des cellules produisant un anticorps humanisé anti peptide-Aβ, ainsi que le procédé de production de cet anticorps comprenant la mise en culture de ces cellules. De telles cellules sont avantageusement issues d'une lignée cellulaire.

La présente demande décrit un médicament comprenant un anticorps humanisé anti peptide-Aβ.

La présente demande décrit un polynucléotide codant pour un polypeptide présentant au moins 80%, 85%, 90%, 95% ou 99% d'identité avec l'une des séquences SEQ ID NO : 2, 4, 6, 8, 22, 24, 26 ou 28.
La présente demande décrit un polynucléotide présentant une séquence ayant au moins 80%, 85%, 90%, 95% ou 99% d'identité avec l'une des séquences SEQ ID NO : 1, 3, 5, 7, 21, 23, 25, ou 27.

La présente demande décrit un vecteur recombinant comprenant un acide nucléique ayant l'une des séquences SEQ ID NO 1, 3, 5, 7, 21, 23, 25, ou 27, ainsi qu'une cellule hôte comprenant ce vecteur.

### Définitions

Une liaison spécifique s'entend d'une différence d'un facteur d'au moins environ 10, 20, 30, 40, 50, ou 100 entre l'intensité de la liaison à un récepteur par rapport à un autre, ici entre la liaison à la forme protofibrillaire du peptide A-β et la liaison aux autres formes du peptide.

Par « épitope » on entend le site de l'antigène auquel se lie l'anticorps. Si l'antigène est un polymère, tel une protéine ou un polysaccharide, l'épitope peut être formé par des résidus contigus ou non-contigus. Ici l'épitope est conformationnel, c'est-à-dire lié à la structure tri-dimensionnelle du peptide A-β protofibrillaire.

Par « forme protofibrillaire » on entend une forme oligomérique de peptides A-β, soluble in vitro et pouvant être isolée comme une entité de poids moléculaire supérieur à 200 kDa, 300 kDa, 400 kDa ou 500 kDa et qui peut fixer des agents tels que la thioflavine-S ou le rouge Congo.

Par « plaque sénile » on entend une plaque composée d'un noyau amyloïde (fixant la thioflavine S ou le rouge Congo) entouré de neurites dystrophiques et d'une réaction de cellules gliales. Les plaques séniles sont retrouvées en particulier chez les patients atteints de la maladie d'Alzheimer, contrairement aux dépôts amyloïdes diffus (ne fixant pas la thioflavine S ou le rouge Congo) qui sont beaucoup plus nombreux mais ne sont pas associés à la maladie.

Un anticorps, aussi appelé immunoglobuline, est composé de deux chaînes lourdes identiques (« CH ») et de deux chaînes légères identiques (« CL ») qui sont liées par un pont disulfure. Chaque chaîne contient une région constante et une région variable. Chaque région variable comprend trois segments appelés « régions déterminant la complémentarité » (« CDRs ») ou « régions hypervariables », qui sont principalement responsables de la liaison à l'épitope d'un antigène.

Le terme « VH » fait référence aux régions variables d'une chaîne lourde d'immunoglobuline d'un anticorps, incluant les chaînes lourdes d'un fragment Fv, scFv, dsFv, Fab, Fab' ou F(ab)'.

Le terme « VL » fait référence aux régions variables d'une chaîne légère d'immunoglobuline d'un anticorps, incluant les chaînes légères d'un fragment Fv, scFv, dsFv, Fab, Fab' ou F(ab)'.

Par « anticorps » on entend également tout fragment fonctionnel d'anticorps: Fab (Fragment antigen binding), Fv, scFv (single chain Fv), Fc (Fragment cristallisable). De préférence, ces fragments fonctionnels seront des fragments de type Fv, scFv, Fab, F(ab') 2, Fab', scFv-Fc, des diabodies, des anticorps multispécifiques (notamment bi-spécifiques), des polypeptides synthétiques contenant les séquences d'un ou de CDRs, qui possèdent généralement la même spécificité de fixation que l'anticorps humanisé dont ils sont issus. Selon la présente invention, des fragments d'anticorps de l'invention peuvent être obtenus à partir des anticorps humanisés par des méthodes telles que la digestion par des enzymes, comme la pepsine ou la papaïne et/ou par clivage des ponts disulfures par réduction chimique.
Les nanobodies rentrent également dans cette définition.

Par « régions CDR ou CDRs », on entend désigner les régions hypervariables des chaînes lourdes et légères des immunoglobulines comme définies par Kabat et al. (Kabat et al., Sequences of proteins of immunological interest, 5t" Ed., U.S. Department of Health and Human Services, NIH, 1991, and later editions). Il existe 3 CDRs de chaîne lourde et 3 CDRs de chaîne légère. Le terme CDR ou CDRs est utilisé ici pour désigner suivant les cas, l'une de ces régions ou plusieurs, voire l'ensemble, de ces régions qui contiennent la majorité des résidus d'acides aminés responsables de la liaison affine de l'anticorps pour l'antigène ou l'épitope qu'il reconnaît. Les régions les plus conservées des domaines variables sont appelés régions ou séquences FR pour « framework » ou régions « charpentes ».

La présente demande décrit des anticorps humanisés.
Par « anticorps humanisé » on entend un anticorps qui contient principalement des séquences immunoglobuline humaines. Ce terme fait généralement référence à une immunoglobuline non humaine qui a été modifiées par incorporation de séquences humaines ou de résidus trouvés dans des séquences humaines.

En général, les anticorps humanisés comprennent un ou typiquement deux domaines variables dans lesquels tout ou partie des régions CDR correspondent à des parties issues de la séquence parente non humaine et dans lesquels tout ou partie des régions FR sont issus d'une séquence immunoglobuline humaine. L'anticorps humanisé peut alors comprendre au moins une portion d'une région constante d'immunoglobuline (Fc), en particulier celle de l'immunoglobuline humaine de référence choisie.
On cherche ainsi à obtenir un anticorps qui soit immunogène a minima chez un humain. Ainsi il est possible qu'un ou deux acides aminés d'un ou plusieurs CDRs soient modifiés par un acide aminé moins immunogène pour l'hôte humain, ceci sans réduire substantiellement la spécificité de liaison de l'anticorps au peptide A-β de haut poids moléculaire. De même, les résidus des régions charpentes peuvent ne pas être humains et il est possible qu'ils ne soient pas modifiés car ils ne contribuent pas au potentiel immunogénique de l'anticorps.

Il existe plusieurs méthodes d'humanisation connues de l'homme du métier pour modifier un anticorps parent non-humain en un anticorps moins immunogène pour l'humain. Une identité globale des séquences avec un anticorps humain n'est pas forcément nécessaire. En effet l'identité globale de séquence n'est pas nécessairement un indicateur prédictif d'une immunogénicité réduite et la modification d'un nombre limité de résidus peut conduire à des anticorps humanisés présentant un potentiel immunogénique très atténué chez l'homme (Molecular Immunology (2007) 44, 1986-1998).

Diverses méthodes sont par exemple l'inclusion de CDRs (grafting) (EPO 0 239 400; WO 91/09967; et U.S. Pat. Nos. 5,530,101 et 5,585,089), le resurfaçage (EPO 0 592 106; EPO 0 519 596; Padlan, 1991, Molec Imm 28(4/5):489-498; Studnicka et al., 1994, Prot Eng 7(6):805-814; et Roguska et al., 1994, PNAS 91:969-973) ou encore le mélange des chaînes (U.S. Pat. No. 5,565,332).

La présente demande décrit en particulier des anticorps humanisés dont les parties variables sont modifiées selon la technologie explicitée dans la demande de brevet internationale WO 2009/032661.
Cette technique utilise notamment une simulation de dynamique moléculaire à partir de modèles tridimensionnels d'anticorps, lesdits modèles étant construits par homologie.

La présente demande décrit aussi toute forme d'anticorps ayant des fonctions effectrices diminuées, comme des immunoglobulines portant des mutations du domaine Fc réduisant son affinité pour les récepteurs du système immunitaire ou comme des nanobodies.

Par « fonctions effectrices » on entend toute fixation du domaine Fc de l'anticorps à des récepteurs ou protéines induisant des réponses immunitaires. La diminution de ces fonctions effectrices permet de diminuer des effets adverses tels que le déclenchement de micro-hémorragies (Racke et al. J Neurosci 2005, 25:629).

L'affinité peut être mesurée par toute technique connue de l'homme du métier. Elle est avantageusement mesurée selon la technique Biostat Speed développée à partir des algorithmes décrits par Ratkovsk DA et Reedy TJ (Biometrics, 1986, 42, 575-82).

Afin de permettre l'expression de chaînes lourdes et/ou chaînes légères de l'anticorps, objet de l'invention, les polynucléotides codant pour lesdites chaînes sont insérés dans des vecteurs d'expression. Ces vecteurs d'expression peuvent être des plasmides, des YACs, des cosmides, des rétrovirus, des épisomes dérivés d'EBV, et tous les vecteurs que l'homme du métier peut juger appropriés à l'expression desdites chaînes.

Ces vecteurs peuvent être utilisés pour transformer des cellules avantageusement issues d'une lignée cellulaire. Une telle lignée cellulaire est encore plus avantageusement issue d'un mammifère.
Elle est avantageusement la lignée CHO ou une lignée dérivée de cette lignée, ou encore la lignée HEK293 ou une lignée dérivée de cette lignée.

La transformation des cellules peut être effectuée par toute méthode connue de l'homme du métier pour introduire des polynucléotides dans une cellule hôte. Une telle méthode peut être la transformation à l'aide de dextrane, la précipitation par du phosphate de calcium, la transfection à l'aide de polybrène, la fusion de protoplastes, l'électroporation, l'encapsulation des polynucléotides dans liposomes, l'injection biolistique et la micro-injection directe d'DNA dans le noyau.

Les anticorps objet de l'invention peuvent être compris dans des compositions pharmaceutiques en vue d'une administration par voie topique, orale, parentérale, intranasale, intraveineuse, intramusculaire, sous-cutanée, intraoculaire, etc. Préférentiellement, les compositions pharmaceutiques contiennent des véhicules pharmaceutiquement acceptables pour une formulation injectable. Il peut s'agir en particulier de solutions salines (phosphate monosodique, disodique, chlorure de sodium, potassium, calcium ou magnésium, etc., ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

A titre d'exemple une composition pharmaceutique comprend (1) un tampon phosphate Dulbecco (pH ∼ 7.4), contenant éventuellement 1 mg/ml à 25 mg/ml de sérum albumine humaine, (2) 0.9% w/v de chlorure de sodium (NaCl)), et (3) 5% (w/v) de dextrose. Elle peut aussi comprendre un antioxydant tel que du tryptamine et un agent stabilisant tel que du Tween 20.

Les pathologies visées peuvent être toutes les maladies liées au dépôt de plaques amyloïdes. En particulier, la pathologie visée est la maladie d'Alzheimer.

Les doses dépendent de l'effet souhaité, de la durée du traitement et de la voie d'administration utilisée ; elles sont généralement comprises entre 5 mg et 1000 mg d'anticorps par jour pour un adulte. De manière générale le médecin déterminera le dosage approprié en fonction du stade de la maladie, de l'âge du patient, de son poids ou de tout autre facteur à prendre en compte en fonction du patient.

La présente invention est illustrée, sans pour autant être limitée, par les exemples qui suivent.

### Brève description des figures :

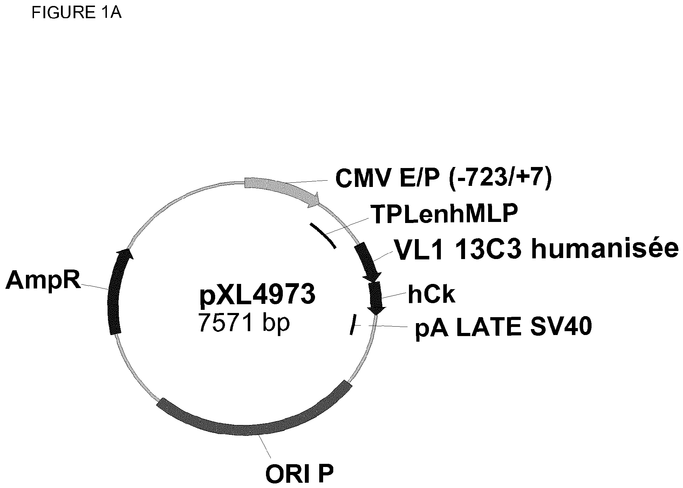

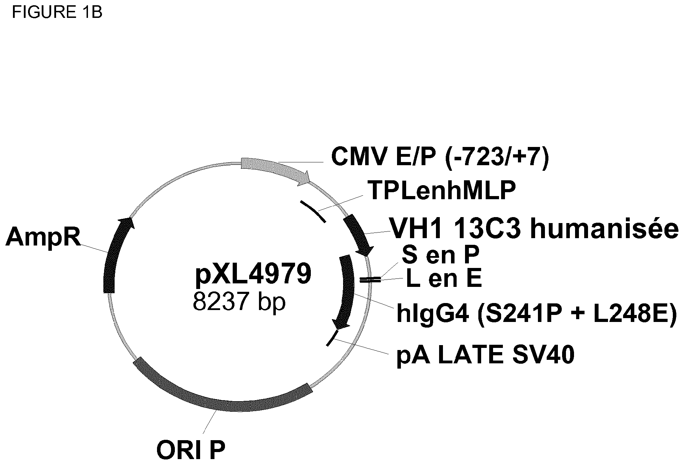

**Fig 1** **A:** Carte du plasmide pXL4973 permettant l'expression de la chaîne légère LC1 de l'anticorps antiAbeta 13C3-VH1VL1.
**Fig 1** **B:** Carte du plasmide pXL4979 permettant l'expression de la chaîne lourde HC1 de l'anticorps antiAbeta 13C3-VH1VL1.
**Fig 2A et 2B** **:** Séparation des protofibrillles et des oligomères de faible masse moléculaire par gel filtration sur Superdex 75 (respectivement à t=0 et à t=16h).
**Fig 3** **:** Détermination de la masse moléculaire des protofibrilles.
**Fig 4** **A, 4B et 4C :** Détermination des affinités des anticorps humanisés (respectivement anticorps LP09027 (4A), LP09026 (4B) et LP09028 (4C)) pour les protofibrilles (moyenne de 3 expériences ± sem).
**Fig 5** **:** Spécificité de l'anticorps humanisé LP09027 vis à vis des fibrilles de Aβ.
**Fig 6A et 6B** **:** Spécificité de l'anticorps humanisé (LP09027) pour les plaques matures séniles respectivement du cortex frontal (6A) et de l'hippocampe (6B) d'une souris. Les flèches indiquent les plaques séniles.

### EXEMPLES:

### Exemple 1 : Obtention des anticorps humanisés

Un anticorps murin 13C3 a été humanisé.

Cet exemple décrit la séquence et l'obtention de l'anticorps humanisé anti-peptide Aβ VH1VL1 (LP09027) par production en expression transitoire dans la lignée mammifère HEK293 nommée FreeStyle 293-F.

Les cDNA codant pour les chaines variables humanisées VL1 et VH1 sont fusionnés aux cDNA codant pour les régions constantes humaines Ckappa et IgG4 respectivement. La séquence de la région constante IgG4 est celle du variant ayant les substitutions S241P and L248E dans la nomenclature de Kabat, afin de diminuer significativement la production de demi-molécules (Angla et al., 1993, Mol. Immunol., 30: 105-108) et les fonctions effectrices (WO 97/09351).

Les séquences nucléiques codant pour CH1 (SEQ ID NO 1) et pour CL1 (SEQ ID NO 3) ont été clonées indépendamment dans le vecteur d'expression pour générer respectivement les plasmides pXL4973 (Fig 1A) et pXL4979 (Fig 1B).

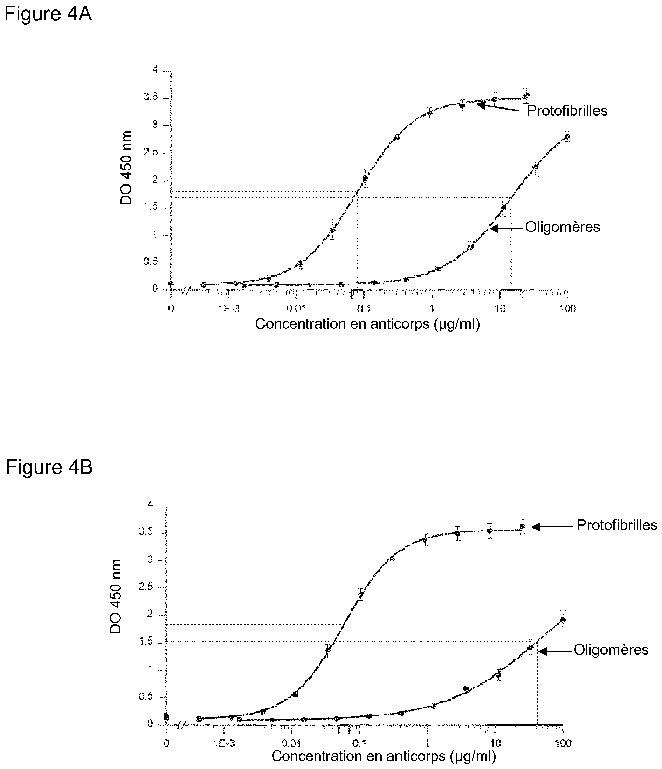

Un lot de l'anticorps est produit par production en expression transitoire dans la lignée FreeStyle 293-F (Invitrogen) après co-transfection des plasmides pXL4973 et pXL4979 selon le protocole décrit par Invitrogen (référence catalogue K9000-01). Ce lot (LP09027) est ensuite purifié par chromatographie d'affinité sur une colonne de gel MabSelect (Amersham) selon les recommandations du fournisseur puis formulé dans le tampon PBS (référence Dulbecco 14190-094) et filtré stérilement (0.2 µm). A partir de 1 L de culture, 33 mg d'anticorps sont obtenus avec une pureté de 97 % par SDS-PAGE dans des conditions dénaturantes et par chromatographie d'exclusion stérique. La masse obtenue par SDS-PAGE dans des conditions dénaturantes et par LC/MS est en accord avec la séquence primaire en acide aminés et la présence d'un N-glycan sur le domaine Fc soit une masse de 23969 Da pour la LC1 et 49650 Da pour la HC1 prenant en compte le N-glycan sous la forme G0F. La masse obtenue par SDS-PAGE dans des conditions non dénaturantes et par chromatographie d'exclusion de taille est en accord avec la structure hétéro-tétramérique de l'anticorps de 150 kDa.(Fig 4A).

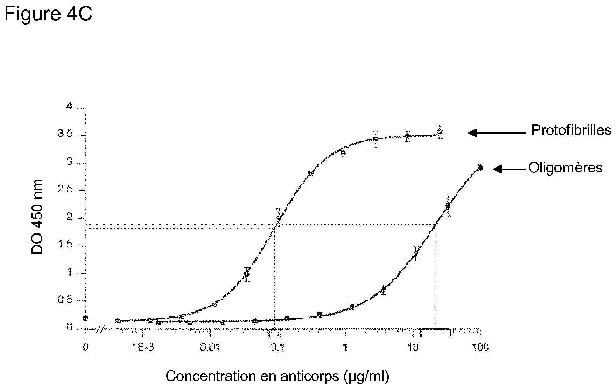

Selon le même procédé, les lots d'anticorps humanisés LP09026 et LP09028 ont été produits à partir des séquences nucléotidiques SEQ ID NO 25 et SEQ ID NO 21 pour LP09026 (Fig 4B), et SEQ ID NO 1 et SEQ ID NO 21 pour LP09028 (Fig 4C).

### Exemple 2 : Préparation des protofibrilles à partir du peptide Aβ (1-42).

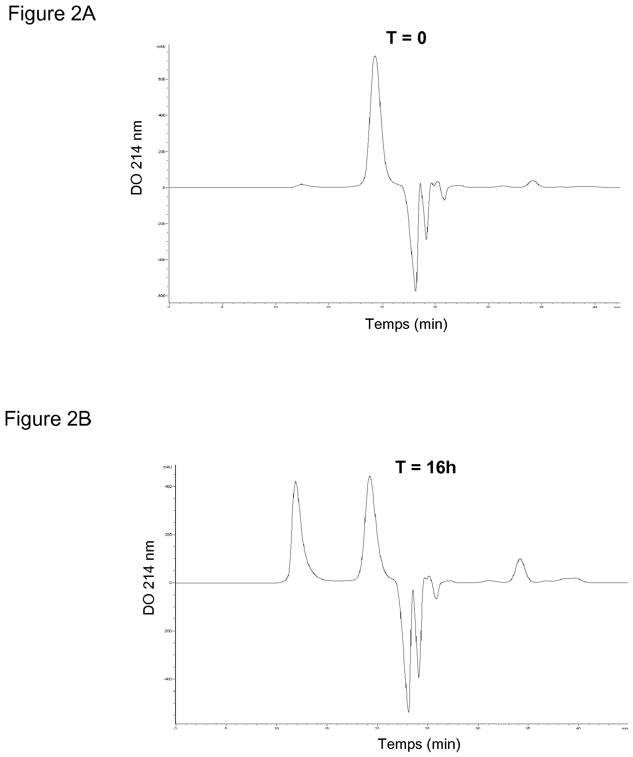

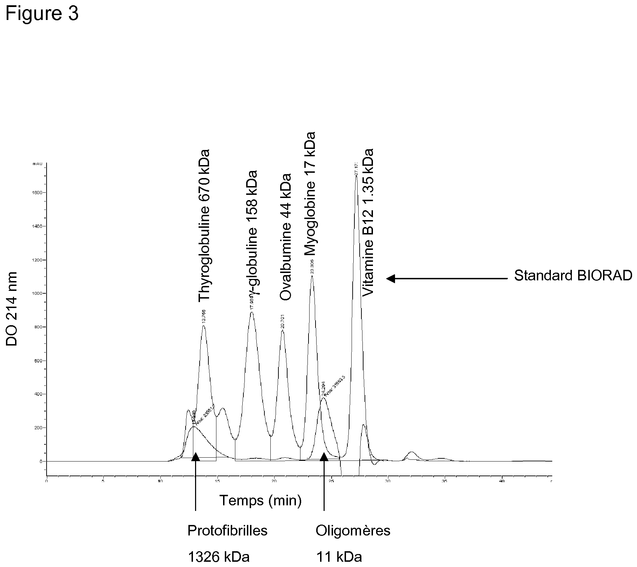

Les protofibrilles ont été préparées à partir du peptide synthétique Aβ (1-42) selon la méthode décrite par Johansson et al. (FEBS, 2006, 2618-2630). Le peptide lyophilisé (Anaspec référence 24224) est mis en solution dans 10mM NaOH à la concentration de 100µM, puis agité pendant 1 mn et incubé sur la glace pendant 10mn. La solution de peptide est ensuite diluée dans le tampon 100mM phosphate de sodium, 200mM NaCl pH=7.4 à une concentration de 50 µM, puis agité 1mn. La préparation est incubée une nuit à 37°C pour obtenir la formation de protofibrilles puis centrifugée à 17900g pendant 15 mn à 16°C pour éliminer les agrégats insolubles. Pour séparer les protofibrilles des formes oligomériques de Aβ de faible masse moléculaire, le surnageant est chargée sur une colonne de gel filtration Superdex 75 équilibrée dans le tampon 50 mM acétate d'ammonium pH=8.5. Les fractions correspondant aux protofibrilles et aux oligomères de faible masse moléculaire sont collectées et stockées à 4°C. La figure 2 montre un profil type de séparation des protofibrilles. La masse moléculaire des protofibrilles est déterminée par gel filtration Superdex200 en utilisant comme marqueurs de masse de moléculaire le kit de calibration Biorad (référence 150-1901). La figure 3 montre que la masse moléculaire des protofibrilles est supérieure à 200KDa.

### Exemple 3 : Spécificité et affinité des anticorps humanisés vis-à-vis des protofibrilles

50µl de protofibrilles et oligomères de faible masse moléculaire à la concentration de 1 µg/ml dans du PBS (Gibco, référence 70011) sont déposés dans les puits d'une plaque ELISA (Nunc, référence 442404) et incubés à 4°C pendant une nuit. Après élimination de l'excès d'antigène, 200 µl de tampon PBS + 5% lait en poudre (poids /volume) sont déposés dans chaque puits pour éliminer les adsorptions non spécifiques et incubés pendant 2h à température ambiante. Les puits sont ensuite lavés 4 fois avec 300 µl de tampon PBS tween 0.02%. 50µl d'une solution d'anticorps primaire (dilution de 3 en 3 dans du PBS Tween à partir d'une concentration de 100µg/ml pour les oligomères et à partir de 25 µg/ml pour les protofibrilles) sont ajoutés à chaque puits et incubés 1h à température ambiante. Les puits sont lavés 4 fois avec 300µl de tampon PBS Tween. L'anticorps secondaire anti-Fc humain couplé à la peroxydase (Goat Anti Human IgG (Fc) peroxidase conjugated, Pierce, référence 31413) dilué au 1/10000 dans du tampon PBS Tween est ajouté à chaque puits et incubé 1h à température ambiante. Après 4 lavages avec 300µl de PBS Tween, 100µl de TMB (Interchim, référence UP664782) sont ajoutés à chaque puits et incubés pendant environ 10mn, puis la réaction est arrêtée avec une solution de HCl 1M (Interchim, référence UPS29590) et les plaques sont lues à une DO mesurée à la longueur d'onde de 450nm. Les EC50 sont déterminées par BioStat Speed. Les résultats obtenus sont indiqués dans le tableau 1 ainsi que dans la figure 4 et montrent la très grande spécificité de l'anticorps pour les protofibrilles par rapport aux oligomères de faible masse moléculaires (facteur 184).

**Tableau 1 :**

| EC50 (µg/ml) | LMW | PF | LMW/PF |
|---|---|---|---|
| LP09026 | 41.4 ± 40.1 | 0.0587 ± 0.004 | 705.3 |
| LP09027 | 14.7 ± 2.7 | 0.0798 ± 0.007 | 184.2 |
| LP09028 | 21.8 ± 5.3 | 0.0892 ± 0.007 | 244.4 |

Le peptide Aβ1-42 lyophilisé (Anaspec référence 24224) est dissous selon les recommandations du fournisseur: 40 µl de NH4OH 1% sont ajoutés à 500 µg d'Aβ1-42. Après complète dissolution, 460 µl de PBS sont ajoutés pour obtenir une concentration de 1 mg/ml. Des aliquots de 10 µl sont préparés et stockés à -80°C.
50µl d'une solution de peptide Aβ1-42 à la concentration de 1µg/ml dans le tampon carbonate (NaHCO₃ 0.025 M (Acros Organics, référence 217120010), Na₂CO₃ 0.025 M (Acros Organics, référence 207810010), pH 9.7 est déposée dans les puits d'une plaque ELISA et incubée pendant la nuit à température ambiante. Comme précédemment les puits sont lavés avec le tampon PBS Tween, incubés en présence de tampon PBS + 5% lait en poudre (poids /volume) et lavés avec le tampon PBS tween. L'anticorps humanisé à la concentration de 0,02 µg/ml est incubé pendant 1h à température ambiante avec une gamme de concentration (à partir de 1 µg/ml) de peptides Aβ1-28 (Bachem, référence H7865), Aβ1-16 ((Anaspec, référence 24225), Aβ25-35 ((Anaspec, référence 24227), des oligomères de faible masse moléculaire ou des protofibrilles préparés comme décrits précédemment. Le mélange anticorps /antigène est ensuite déposé dans chaque puits et la plaque de microtitration incubée pendant 1h à température ambiante. L'anticorps libre non complexé est déterminé selon le même protocole ELISA que décrit précédemment. Ces expériences de compétition montrent que seules les protofibrilles avec une affinité beaucoup plus grande que les oligomères de faible masse moléculaire sont capables de neutraliser l'anticorps humanisé en l'empêchant d'interagir avec le pepdie Aβ1-42, aucun des peptides n'est capable de neutraliser l'anticorps.

### Exemple 4 : Spécificité de l'anticorps humanisé LP09027 vis à vis des fibrilles de Aβ1-42

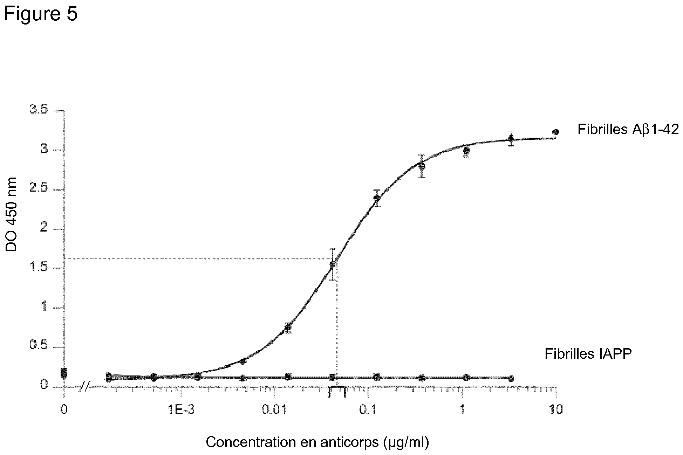

Le peptide Aβ1-42 (Anaspec, 20276) est dissous dans 200 µl de NaOH 10 mM à une concentration de 5 mg/ml. Le peptide IAPP (Anaspec, 60804) est dilué dans 200 µl de DMSO 50% à une concentration de 5 mg/ml. 100 µl de chaque préparation sont dilués dans 400 µl de PBS 1.25X. La concentration finale des peptides est de 1 mg/ml dans 500 µl. Les échantillons sont incubés 72h à 37°C. Après l'incubation, les échantillons sont centrifugés à 17900g pendant 30 minutes à 4°C. Le surnageant est éliminé et le culot est lavé 3 fois avec du PBS 1X. Après le dernier lavage, le culot de fibrilles est repris dans 150 µl de PBS. Pour contrôler la présence de fibrilles de type amyloïde, un test de fluorescence de la thioflavine T (Anaspec, 88306) est réalisé. 20 µl de thioflavine T (20 µM final), 10 µl de l'échantillon et 70 µl de PBS 1X (volume final 100 µl) sont mélangés dans un puit d'une plaque noire (Corning, 3792). La thioflavine T est excitée à 450 nm et, en présence de structure de type amyloïde, émet une fluorescence à 482 nm. 50µl de fibrilles d'Aβ1-42 à 1 µg/ml et IAPP à 0.5 µg/ml sont déposés dans chaque puits d'une plaque de microtitration. Le protocole ELISA est appliqué en utilisant des dilutions en série de l'anticorps humanisé à partir de 10 µg/ml. La figure 5 montre que l'anticorps humanisé LP09027 reconnait spécifiquement les fibrilles de Aβ1-42 mais pas celles d'IAPP.

### Exemple 5: Spécificité de l'anticorps humanisé LP09027 pour les plaques séniles matures et non pour les plaques diffuses

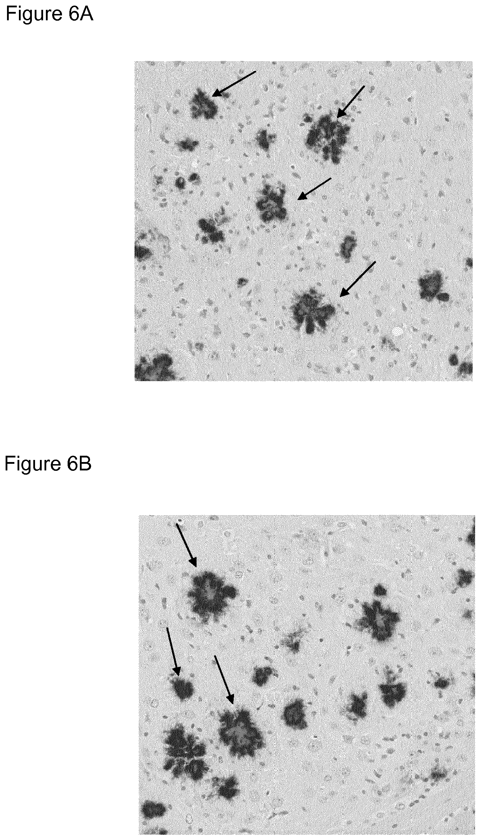

L'anticorps humanisé (LP09027) conjugué avec la digoxygenine (digoxigenin-3-O-methylcarbonyl-ε-aminocaproic acid-N-hydroxysuccinimide ester: Roche 11333054001 ; 11418165001) a été utilisé en Immunohistochimie (Robot Ventana) sur des coupes de cerveaux de souris APP PS1 (modèle Alzheimer décrit par Schmitz C. et al., Am. J. Pathol, 2004, 164, 1495-1502)) ainsi que des coupes de cerveaux humains (cortex cérébral) provenant de patients atteints de la maladie d'Alzheimer. Les échantillons ont été préalablement fixés dans le formol et inclus en paraffine.
Les résultats obtenus chez la souris (figures 6A et 6B) montrent clairement que l'anticorps humanisé reconnaît exclusivement les plaques séniles matures et denses, mais pas les dépôts diffus du peptide Aβ.
Ces données sont en corrélation avec les propriétés de cet anticorps qui est spécifique de la forme Abeta protofibrillaire et ne reconnaît donc pas les formes soluble, mono-ou oligomériques de ce peptide.

**Tableau 2 :**

| | **Séquences nucléotidiques** | **Séquences protéiques** |
|---|---|---|
| **Anticorps 1 VH1VL1** | | |
| VH₁ + CH₁ | SEQ ID NO 1 | SEQ ID NO 2 |
| VL₁ + CL₁ | SEQ ID NO 3 | SEQ ID NO 4 |
| VH₁ | SEQ ID NO 5 | SEO ID NO 6 |
| VL₁ | SEQ ID NO 7 | SEQ ID NO 8 |
| CDR VH₁ | SEQ ID NO 9, 11, 13 | SEQ ID NO 10, 12, 14 |
| CDR VL₁ | SEQ ID NO 15, 17, 19 | SEQ ID NO 16, 18, 20 |

| **Anticorps 2 VH1 VL2** | | |
|---|---|---|
| VH₁ + CH₁ | SEQ ID NO 1 | SEQ ID NO 2 |
| VL₂ + CL₂ | SEQ ID NO 21 | SEQ ID NO 22 |
| VH₁ | SEQ ID NO 5 | SEQ ID NO 6 |
| VL₂ | SEQ ID NO 23 | SEQ ID NO 24 |
| CDR VH₁ | SEQ ID NO 9, 11, 13 | SEQ ID NO 10, 12, 14 |
| CDR VL₂ | SEQ ID NO 31, 17, 19 | SEQ ID NO 32, 18, 20 |

| **Anticorps 3 VH2 VL2** | | |
|---|---|---|
| VH₂ + CH₂ | SEQ ID NO 25 | SEQ ID NO 26 |
| VL₂ + CL₂ | SEQ ID NO 21 | SEQ ID NO 22 |
| VH₂ | SEQ ID NO 27 | SEQ ID NO 28 |
| VL₂ | SEQ ID NO 23 | SEQ ID NO 24 |
| CDR VH₂ | SEQ ID NO 9, 11, 29 | SEQ ID NO 10, 12, 30 |
| CDR VL₂ | SEQ ID NO 31, 17, 19 | SEQ ID NO 32, 18, 20 |

## Revendications

1. Anticorps humanisé spécifique de la forme protofibrillaire du peptide A-β, **caractérisé en ce que**:
- ledit anticorps a des fonctions effectrices diminuées,
- la partie variable de sa chaîne lourde comprend une séquence présentant au moins 90% d'identité avec la séquence SEQ ID NO: 6 ou SEQ ID NO: 28,
- la partie variable de sa chaîne légère comprend une séquence présentant au moins 90% d'identité avec la séquence SEQ ID NO: 8 ou SEQ ID NO: 24,
- ledit anticorps a une affinité pour la forme protofibrillaire du peptide Aβ au moins 100 fois supérieure à son affinité pour les autres formes de ce peptide, l'affinité étant l'EC50 mesurée par ELISA, et
- ledit anticorps se lie aux peptides A-β agrégés en plaques séniles et non aux dépôts diffus de peptides A-β.

2. Anticorps selon la revendication 1, **caractérisé en ce que** ledit anticorps est une immunoglobuline portant des mutations du domaine Fc réduisant son affinité pour les récepteurs du système immunitaire.

3. Anticorps selon la revendication 1 ou 2, **caractérisé en ce que** ledit anticorps est une immunoglobuline G 4 dont le domaine Fc a subi des mutations diminuant l'activité effectrice.

4. Anticorps selon l'une des revendications précédentes, **caractérisé en ce qu'**il comprend au moins un CDR codé par une séquence nucléotidique présentant une séquence identique à l'une des séquences SEQ ID NO: 9, 11, 13, 15, 17 et 19, ou par des séquences différant respectivement par 1, 2, 3, 4 ou 5 nucléotides de ces séquences.

5. Anticorps selon l'une des revendications précédentes, **caractérisé en ce qu'**il comprend au moins un CDR présentant une séquence identique à l'une des séquences SEQ ID NO: 10, 12, 14, 16, 18 et 20.

6. Anticorps selon l'une des revendications précédentes, **caractérisé en ce qu'**il comprend au moins un CDR dont la séquence diffère de un à deux acides aminés par rapport à l'une des séquences SEQ ID NO: 10, 12, 14, 16, 18 et 20, pour autant que l'anticorps garde sa spécificité de liaison.

7. Anticorps selon l'une des revendications précédentes, **caractérisé en ce qu'**il comprend les CDR codés par (i) les séquences nucléotidiques SEQ ID NO: 9, 11, 13, 15, 17 et 19, (ii) les séquences nucléotidiques SEQ ID NO 9, 11, 13, 31, 17 et 19, (iii) les séquences nucléotidiques SEQ ID NO: 9, 11, 29, 31, 17 et 19, ou par des séquences différant respectivement par 1, 2, 3, 4 ou 5 nucléotides de ces séquences.

8. Anticorps selon l'une des revendications précédentes, **caractérisé en ce qu'**il comprend les CDR de séquence SEQ ID NO: 10, 12, 14, 16, 18 et 20, les CDR de séquence SEQ ID NO: 10, 12, 14, 32, 18 et 20, ou les CDR de séquence SEQ ID NO : 10, 12, 30, 32, 18 et 20.

9. Anticorps selon l'une des revendications précédentes, **caractérisé en ce que** :
- la partie variable de sa chaîne lourde est codée par une séquence présentant au moins 80% d'identité avec l'une des séquences SEQ ID NO: 5 et SEQ ID NO: 27,
- la partie variable de sa chaîne légère est codée par une séquence présentant au moins 80% d'identité avec l'une des séquences SEQ ID NO: 7 et SEQ ID NO 23,
- il comprend une chaîne lourde codée par une séquence présentant au moins 80% d'identité avec l'une des séquences nucléotidiques SEQ ID NO: 1 et SEQ ID NO: 25 et/ou une chaîne lourde présentant au moins 80% d'identité avec l'une des séquences polypeptidiques SEQ ID NO: 2 et SEQ ID NO: 26, et/ou
- il comprend une chaîne légère codée par une séquence présentant au moins 80% d'identité avec l'une des séquences nucléotidiques SEQ ID NO: 3 et SEQ ID NO: 21 et/ou une chaîne légère présentant au moins 80% d'identité avec l'une des séquences polypeptidiques SEQ ID NO: 4 et SEQ ID NO: 22.

10. Anticorps selon l'une des revendications précédentes, **caractérisé en ce que** :
- il comprend des séquences codées par (i) les séquences nucléotidiques SEQ ID NO: 5 et 7, (ii) les séquences nucléotidiques SEQ ID NO: 5 et 23 ou (iii) les séquences nucléotidiques SEQ ID NO: 27 et 23, et/ou des séquences codées par (i) les séquences nucléotidiques SEQ ID NO: 1 et 3, (ii) les séquences nucléotidiques SEQ ID NO: 1 et 21 ou (iii) les séquences nucléotidiques SEQ ID NO: 25 et 21, et/ou
- sa séquence comprend les séquences polypeptidiques (i) SEQ ID NO: 6 et 8, (ii) SEQ ID NO: 6 et 24 ou (iii) SEQ ID NO: 28 et 24, et/ou les séquences polypeptidiques (i) SEQ ID NO: 2 et 4, (ii) SEQ ID NO: 2 et 22 ou (iii) SEQ ID NO: 26 et 22.

11. Anticorps selon l'une quelconque des revendications précédentes, **caractérisé en ce qu'**il induit une diminution des plaques amyloïdes.

12. Anticorps selon l'une des revendications 1 à 11, pour utilisation dans le traitement des maladies associées aux troubles neuro-dégénératifs.

13. Anticorps pour utilisation selon la revendication 12, dans le traitement de la maladie d'Alzheimer.

14. Composition pharmaceutique comprenant un anticorps selon l'une quelconque des revendications 1 à 11 et des excipients.

15. Cellule produisant un anticorps selon l'une des revendications 1 à 11.

16. Procédé de production d'un anticorps selon l'une quelconque des revendications 1 à 11, **caractérisé en ce qu'**il comprend la mise en culture de cellules selon la revendication 15.

17. Anticorps selon l'une quelconque des revendications 1 à 11 pour utilisation en tant que médicament.

18. Polynucléotide exprimant l'anticorps selon l'une des revendications 1 à 11, **caractérisé en ce que** ledit polynucléotide code pour un polypeptide présentant au moins 90% d'identité avec l'une des séquences SEQ ID NOS : 2, 4, 6, 8, 22, 24, 26 ou 28, ou présente une séquence présentant au moins 90% d'identité avec l'une des séquences SEQ ID NOS : 1, 3, 5, 7, 21, 23, 25, ou 27.

19. Vecteur recombinant comprenant un acide nucléique selon la revendication 18.

20. Cellule hôte comprenant un vecteur selon la revendication 19.

## Patentansprüche

1. Humanisierter Antikörper, der für die Protofibrillenform des Peptids A-β spezifisch ist, **dadurch gekennzeichnet, dass**
- der Antikörper verringerte Effektorfunktionen besitzt,
- der variable Teil seiner schweren Kette eine Sequenz umfasst, die mindestens 90% Identität mit der Sequenz SEQ ID NO: 6 oder SEQ ID NO: 28 aufweist,
- der variable Teil seiner leichten Kette eine Sequenz umfasst, die mindestens 90% Identität mit der Sequenz SEQ ID NO: 8 oder SEQ ID NO: 24 aufweist,
- der Antikörper eine Affinität für die Protofibrillenform des Peptids A-β besitzt, die mindestens 100-fach höher als seine Affinität für die anderen Formen dieses Peptids ist, wobei die Affinität die mittels ELISA gemessene EC50 ist, und
- der Antikörper an die A-β-Peptide, die in senilen Plaques aggregiert sind, und nicht an die diffusen Ablagerungen von A-β-Peptiden bindet.

2. Antikörper nach Anspruch 1, **dadurch gekennzeichnet, dass** der Antikörper ein Immunglobulin mit Mutationen in der Fc-Domäne ist, die seine Affinität zu den Rezeptoren des Immunsystems verringern.

3. Antikörper nach Anspruch 1 oder 2, **dadurch gekennzeichnet, dass** der Antikörper ein Immunglobulin G4 ist, dessen Fc-Domäne Mutationen unterzogen wurde, die die Effektoraktivität verringern.

4. Antikörper nach einem der vorhergehenden Ansprüche, **dadurch gekennzeichnet, dass** er mindestens eine CDR umfasst, die von einer Nukleotidsequenz, die eine mit einer der Sequenzen SEQ ID NO: 9, 11, 13, 15, 17 und 19 identische Sequenz aufweist, oder von Sequenzen, die sich jeweils um 1, 2, 3, 4 oder 5 Nukleotide von diesen Sequenzen unterscheiden, codiert wird.

5. Antikörper nach einem der vorhergehenden Ansprüche, **dadurch gekennzeichnet, dass** er mindestens eine CDR umfasst, die eine mit einer der Sequenzen SEQ ID NO: 10, 12, 14, 16, 18 und 20 identische Sequenz aufweist.

6. Antikörper nach einem der vorhergehenden Ansprüche, **dadurch gekennzeichnet, dass** er mindestens eine CDR umfasst, deren Sequenz sich um ein bis zwei Aminosäuren von einer der Sequenzen SEQ ID NO: 10, 12, 14, 16, 18 und 20 unterscheidet, insoweit als der Antikörper seine Bindungsspezifität beibehält.

7. Antikörper nach einem der vorhergehenden Ansprüche, **dadurch gekennzeichnet, dass** er die CDR umfasst, die von (i) den Nukleotidsequenzen SEQ ID NO: 9, 11, 13, 15, 17 und 19, (ii) den Nukleotidsequenzen SEQ ID NO: 9, 11, 13, 31, 17 und 19, (iii) den Nukleotidsequenzen SEQ ID NO: 9, 11, 29, 31, 17 und 19 oder von Sequenzen, die sich jeweils um 1, 2, 3, 4 oder 5 Nukleotide von diesen Sequenzen unterscheiden, codiert werden.

8. Antikörper nach einem der vorhergehenden Ansprüche, **dadurch gekennzeichnet, dass** er die CDR der Sequenz SEQ ID NO: 10, 12, 14, 16, 18 und 20, die CDR der Sequenz SEQ ID NO: 10, 12, 14, 32, 18 und 20 oder die CDR der Sequenz SEQ ID NO: 10, 12, 30, 32, 18 und 20 umfasst.

9. Antikörper nach einem der vorhergehenden Ansprüche, **dadurch gekennzeichnet, dass**:
- der variable Teil seiner schweren Kette von einer Sequenz codiert wird, die mindestens 80% Identität mit einer der Sequenzen SEQ ID NO: 5 und SEQ ID NO: 27 aufweist,
- der variable Teil seiner leichten Kette von einer Sequenz codiert wird, die mindestens 80% Identität mit einer der Sequenzen SEQ ID NO: 7 und SEQ ID NO: 23 aufweist,
- er eine schwere Kette, die von einer Sequenz codiert wird, die mindestens 80% Identität mit einer der Nukleotidsequenzen SEQ ID NO: 1 und SEQ ID NO: 25 aufweist, und/oder eine schwere Kette, die mindestens 80% Identität mit einer der Polypeptidsequenzen SEQ ID NO: 2 und SEQ ID NO: 26 aufweist, umfasst, und/oder
- er eine leichte Kette, die von einer Sequenz codiert wird, die mindestens 80% Identität mit einer der Nukleotidsequenzen SEQ ID NO: 3 und SEQ ID NO: 21 aufweist, und/oder eine leichte Kette, die mindestens 80% Identität mit einer der Polypeptidsequenzen SEQ ID NO: 4 und SEQ ID NO: 22 aufweist, umfasst.

10. Antikörper nach einem der vorhergehenden Ansprüche, **dadurch gekennzeichnet, dass**:
- er Sequenzen umfasst, die von (i) den Nukleotidsequenzen SEQ ID NO: 5 und 7, (ii) den Nukleotidsequenzen SEQ ID NO: 5 und 23 oder (iii) den Nukleotidsequenzen SEQ ID NO: 27 und 23 codiert werden, und/oder Sequenzen, die von (i) den Nukleotidsequenzen SEQ ID NO: 1 und 3, (ii) den Nukleotidsequenzen SEQ ID NO: 1 und 21 oder (iii) den Nukleotidsequenzen SEQ ID NO: 25 und 21 codiert werden, und/oder
- seine Sequenz die Polypeptidsequenzen (i) SEQ ID NO: 6 und 8, (ii) SEQ ID NO: 6 und 24 oder (iii) SEQ ID NO: 28 und 24, und/oder die Polypeptidsequenzen (i) SEQ ID NO: 2 und 4, (ii) SEQ ID NO: 2 und 22 oder (iii) SEQ ID NO: 26 und 22 umfasst.

11. Antikörper nach einem der vorhergehenden Ansprüche, **dadurch gekennzeichnet, dass** er eine Verringerung der Amyloidplaques induziert.

12. Antikörper nach einem der Ansprüche 1 bis 11 für die Verwendung bei der Behandlung von Krankheiten, die mit neurodegenerativen Störungen in Zusammenhang stehen.

13. Antikörper für die Verwendung nach Anspruch 12 bei der Behandlung von Alzheimer-Krankheit.

14. Pharmazeutische Zusammensetzung, die einen Antikörper nach einem der Ansprüche 1 bis 11 und Exzipienten umfasst.

15. Zelle, die einen Antikörper nach einem der Ansprüche 1 bis 11 herstellt.

16. Verfahren zur Herstellung eines Antikörpers nach einem der Ansprüche 1 bis 11, **dadurch gekennzeichnet, dass** es das Kultivieren von Zellen nach Anspruch 15 umfasst.

17. Antikörper nach einem der Ansprüche 1 bis 11 für die Verwendung als Arzneimittel.

18. Polynukleotid, das den Antikörper nach einem der Ansprüche 1 bis 11 exprimiert, **dadurch gekennzeichnet, dass** das Polynukleotid für ein Polypeptid codiert, das mindestens 90% Identität mit einer der Sequenzen SEQ ID NO: 2, 4, 6, 8, 22, 24, 26 oder 28 aufweist, oder eine Sequenz aufweist, die mindestens 90% Identität mit einer der Sequenzen SEQ ID NO: 1, 3, 5, 7, 21, 23, 25 oder 27 aufweist.

19. Rekombinanter Vektor, der eine Nukleinsäure nach Anspruch 18 umfasst.

20. Wirtszelle, die einen Vektor nach Anspruch 19 umfasst.

## Claims

1. A humanized antibody specific for the protofibrillar form of the A-β peptide, **characterized in that**:
- said antibody has reduced effector functions,
- the variable part of its heavy chain comprises a sequence having at least 90% identity with sequence SEQ ID NO: 6 or SEQ ID NO: 28,
- the variable part of its light chain comprises a sequence having at least 90% identity with sequence SEQ ID NO: 8 or SEQ ID NO: 24,
- said antibody has an affinity for the protofibrillar form of the Aβ peptide at least 100 times greater than its affinity for the other forms of this peptide, the affinity being EC50 measured by ELISA; and
- said antibody binds to the A-β peptides aggregated in senile plaques and not to the diffuse deposits of A-β peptides.

2. The antibody according to claim 1, **characterized in that** said antibody is an immunoglobulin bearing mutations of the Fc domain reducing its affinity for the receptors of the immune system.

3. The antibody according to claim 1 or 2, **characterized in that** said antibody is an immunoglobulin G 4 whose Fc domain has undergone mutations reducing the effector activity.

4. The antibody according to any one of the preceding claims, **characterized in that** it comprises at least one CDR encoded by a nucleotide sequence having a sequence identical to one of the sequences SEQ ID NO: 9, 11, 13, 15, 17 and 19, or by sequences differing respectively by 1, 2, 3, 4 or 5 nucleotides from these sequences.

5. The antibody according to any one of the preceding claims, **characterized in that** it comprises at least one CDR having a sequence identical to one of the sequences SEQ ID NO: 10, 12, 14, 16, 18 and 20.

6. The antibody according to any one of the preceding claims, **characterized in that** it comprises at least one CDR whose sequence differs by one to two amino acids relative to one of the sequences SEQ ID NO: 10, 12, 14, 16, 18 and 20, inasmuch as the antibody maintains its binding specificity.

7. The antibody according to any one of the preceding claims, **characterized in that** it comprises the CDRs encoded by (i) the nucleotide sequences SEQ ID NO: 9, 11, 13, 15, 17 and 19, (ii) the nucleotide sequences SEQ ID NO: 9, 11, 13, 31, 17 and 19, (iii) the nucleotide sequences SEQ ID NO: 9, 11, 29, 31, 17 and 19, or by sequences differing respectively by 1, 2, 3, 4 or 5 nucleotides from these sequences.

8. The antibody according to any one of the preceding claims, **characterized in that** it comprises the CDRs of sequence SEQ ID NO: 10, 12, 14, 16, 18 and 20, the CDRs of sequence SEQ ID NO: 10, 12, 14, 32, 18 and 20, or the CDRs of sequence SEQ ID NO: 10, 12, 30, 32, 18 and 20.

9. The antibody according to any one of the preceding claims, **characterized in that**:
- the variable part of its heavy chain is encoded by a sequence having at least 80% identity with one of the sequences SEQ ID NO: 5 and SEQ ID NO:27,
- the variable part of its light chain is encoded by a sequence having at least 80% identity with one of the sequences SEQ ID NO: 7 and SEQ ID NO: 23,
- it comprises a heavy chain encoded by a sequence having at least 80% identity with one of the nucleotide sequences SEQ ID NO: 1 and SEQ ID NO: 25 and/or a heavy chain having at least 80% identity with one of the polypeptide sequences SEQ ID NO: 2 and SEQ ID NO: 26, and/or
- it comprises a light chain encoded by a sequence having at least 80% identity with one of the nucleotide sequences SEQ ID NO: 3 and SEQ ID NO: 21 and/or a light chain having at least 80% identity with one of the polypeptide sequences SEQ ID NO: 4 and SEQ ID NO: 22.

10. The antibody according to any one of the preceding claims, **characterized in that**:
- it comprises sequences encoded by (i) the nucleotide sequences SEQ ID NO: 5 and 7, (ii) the nucleotide sequences SEQ ID NO: 5 and 23 or (iii) the nucleotide sequences SEQ ID NO: 27 and 23, and/or sequences encoded by (i) the nucleotide sequences SEQ ID NO: 1 and 3, (ii) the nucleotide sequences SEQ ID NO: 1 and 21 or (iii) the nucleotide sequences SEQ ID NO: 25 and 21, and/or
- its sequence comprises the polypeptide sequences (i)SEQ ID NO: 6 and 8, (ii)SEQ ID NO: 6 and 24, or (iii)SEQ ID NO: 28 and 24, and/or the polypeptide sequences(i)SEQ ID NO: 2 and 4, (ii)SEQ ID NO: 2 and 22, (iii)SEQ ID NO: 26 and 22.

11. The antibody according to any one of the preceding claims, **characterized in that** it induces a reduction of the amyloid plaques.

12. The antibody according to any one of claims 1 to 11, for use in the treatment of diseases associated with neurodegenerative disorders.

13. The antibody for use according to claim 12, in the treatment of Alzheimer's disease.

14. A pharmaceutical composition comprising an antibody according to any one of claims 1 to 11 and excipients.

15. A cell producing an antibody according to any one of claims 1 to 11.

16. A method of production of an antibody according to any one of claims 1 to 11, **characterized in that** it comprises the culturing of cells according to claim 15.

17. The antibody according to any one of claims 1 to 11 for use as a medicament.

18. A polynucleotide expressing the antibody according to any one of claims 1 to 11, **characterized in that** said polynucleotide encodes for a polypeptide having at least 90% identity with one of the sequences SEQ ID NOS: 2, 4, 6, 8, 22, 24, 26 or 28, or has a sequence having at least 90% identity with one of the sequences SEQ ID NOS: 1, 3, 5, 7, 21, 23, 25 or 27.

19. A recombinant vector comprising a nucleic acid according to claim 18.

20. A host cell comprising a vector according to claim 19.


### SEQUENCE LISTING

<110> SANOFI-AVENTIS
<120> Anticorps humanisés spécifiques de la forme protofibrillaire du peptide béta-amyloïde
<130> FR2009-054
<160> 32
<170> PatentIn version 3.3
<210> 1
   <211> 1326
   <212> DNA
   <213> Artificial
<220>
   <223> Séquence humanisée
<220>
   <221> CDS
   <222> (1)..(1326)
<400> 1
   gag gtc cag ctg cag cag tct ggg cct gag gtg gtg aag cct ggg gtc        48
   Glu Val Gln Leu Gln Gln Ser Gly Pro Glu Val Val Lys Pro Gly Val
   1               5                   10                  15
   tca gtg aag att tcc tgc aag ggt tcc ggc tac aca ttc act gat tat        96
   Ser Val Lys Ile Ser Cys Lys Gly Ser Gly Tyr Thr Phe Thr Asp Tyr
               20                  25                  30
   gct atg cac tgg gtg aag cag agt cct ggc aag agt ctg gag tgg att       144
   Ala Met His Trp Val Lys Gln Ser Pro Gly Lys Ser Leu Glu Trp Ile
           35                  40                  45
   gga gtt att agt act aag tat ggt aag aca aac tac aac ccc agc ttt       192
   Gly Val Ile Ser Thr Lys Tyr Gly Lys Thr Asn Tyr Asn Pro Ser Phe
       50                  55                  60
   cag ggc cag gcc aca atg act gtt gac aaa tcc tcc agc aca gcc tat       240
   Gln Gly Gln Ala Thr Met Thr Val Asp Lys Ser Ser Ser Thr Ala Tyr
   65                  70                  75                  80
   atg gag ctt gcc agc ttg aag gcc tcc gat tct gcc atc tat tac tgt       288
   Met Glu Leu Ala Ser Leu Lys Ala Ser Asp Ser Ala Ile Tyr Tyr Cys
                   85                  90                  95
   gca aga ggg gac gat ggt tat tcc tgg ggt caa gga acc tca gtc acc       336
   Ala Arg Gly Asp Asp Gly Tyr Ser Trp Gly Gln Gly Thr Ser Val Thr
               100                 105                 110
   gtc tcc agc gct tct acc aag ggc cct tcc gtg ttc cct ctg gcc cct       384
   Val Ser Ser Ala Ser Thr Lys Gly Pro Ser Val Phe Pro Leu Ala Pro
           115                 120                 125
   tgc tcc cgg tcc acc tcc gag tcc acc gcc gct ctg ggc tgc ctg gtg       432
   Cys Ser Arg Ser Thr Ser Glu Ser Thr Ala Ala Leu Gly Cys Leu Val
       130                 135                 140
   aag gac tac ttc cct gag cct gtg acc gtg tcc tgg aac tct ggc gcc      480
   Lys Asp Tyr Phe Pro Glu Pro Val Thr Val Ser Trp Asn Ser Gly Ala
   145                 150                 155                 160
   ctg acc tcc ggc gtg cac acc ttc cct gcc gtg ctg cag tcc tcc ggc      528
   Leu Thr Ser Gly Val His Thr Phe Pro Ala Val Leu Gln Ser Ser Gly
                   165                 170                 175
   ctg tac tcc ctg tcc tcc gtg gtg acc gtg cct tcc tcc tcc ctg ggc      576
   Leu Tyr Ser Leu Ser Ser Val Val Thr Val Pro Ser Ser Ser Leu Gly
               180                 185                 190
   acc aag acc tac acc tgt aac gtg gac cac aag cct tcc aac acc aag      624
   Thr Lys Thr Tyr Thr Cys Asn Val Asp His Lys Pro Ser Asn Thr Lys
           195                 200                 205
   gtg gac aag cgg gtg gag tcc aag tac ggc cct cct tgc cct ccc tgc      672
   Val Asp Lys Arg Val Glu Ser Lys Tyr Gly Pro Pro Cys Pro Pro Cys
       210                 215                 220
   cct gcc cct gag ttc gag ggc gga cct agc gtg ttc ctg ttc cct cct      720
   Pro Ala Pro Glu Phe Glu Gly Gly Pro Ser Val Phe Leu Phe Pro Pro
   225                 230                 235                 240
   aag cct aag gac acc ctg atg atc tcc cgg acc cct gag gtg acc tgt      768
   Lys Pro Lys Asp Thr Leu Met Ile Ser Arg Thr Pro Glu Val Thr Cys
                   245                 250                 255
   gtg gtg gtg gac gtg tcc cag gag gac cct gag gtc cag ttc aac tgg      816
   Val Val Val Asp Val Ser Gln Glu Asp Pro Glu Val Gln Phe Asn Trp
               260                 265                 270
   tac gtg gac ggc gtg gag gtg cac aac gcc aag acc aag cct cgg gag      864
   Tyr Val Asp Gly Val Glu Val His Asn Ala Lys Thr Lys Pro Arg Glu
           275                 280                 285
   gag cag ttc aat tcc acc tac cgg gtg gtg tct gtg ctg acc gtg ctg      912
   Glu Gln Phe Asn Ser Thr Tyr Arg Val Val Ser Val Leu Thr Val Leu
       290                 295                 300
   cac cag gac tgg ctg aac ggc aaa gaa tac aag tgt aag gtc tcc aac      960
   His Gln Asp Trp Leu Asn Gly Lys Glu Tyr Lys Cys Lys Val Ser Asn
   305                 310                 315                 320
   aag ggc ctg ccc tcc tcc atc gag aaa acc atc tcc aag gcc aag ggc     1008
   Lys Gly Leu Pro Ser Ser Ile Glu Lys Thr Ile Ser Lys Ala Lys Gly
                   325                 330                 335
   cag cct agg gag cct cag gtg tac acc ctg cct cct agc cag gaa gag     1056
   Gln Pro Arg Glu Pro Gln Val Tyr Thr Leu Pro Pro Ser Gln Glu Glu
               340                 345                 350
   atg acc aag aac cag gtg tcc ctg acc tgt ctg gtg aag ggc ttc tac     1104
   Met Thr Lys Asn Gln Val Ser Leu Thr Cys Leu Val Lys Gly Phe Tyr
           355                 360                 365
   cct tcc gac atc gcc gtg gag tgg gag tcc aac ggc cag cct gag aac     1152
   Pro Ser Asp Ile Ala Val Glu Trp Glu Ser Asn Gly Gln Pro Glu Asn
       370                 375                 380
   aac tac aag acc acc cct cct gtg ctg gac tcc gac ggc tcc ttc ttc     1200
   Asn Tyr Lys Thr Thr Pro Pro Val Leu Asp Ser Asp Gly Ser Phe Phe
   385                 390                 395                 400
   ctg tac tcc agg ctg acc gtg gac aag tcc cgg tgg cag gag ggc aac      1248
   Leu Tyr Ser Arg Leu Thr Val Asp Lys Ser Arg Trp Gln Glu Gly Asn
                   405                 410                 415
   gtc ttt tcc tgc tcc gtg atg cac gag gcc ctg cac aac cac tac acc      1296
   Val Phe Ser Cys Ser Val Met His Glu Ala Leu His Asn His Tyr Thr
               420                 425                 430
   cag aag tcc ctg tcc ctg tct ctg ggc tga                              1326
   Gln Lys Ser Leu Ser Leu Ser Leu Gly
           435                 440
<210> 2
   <211> 441
   <212> PRT
   <213> Artificial
<220>
   <223> Synthetic Construct
<400> 2
   Glu Val Gln Leu Gln Gln Ser Gly Pro Glu Val Val Lys Pro Gly Val
   1               5                   10                  15
   Ser Val Lys Ile Ser Cys Lys Gly Ser Gly Tyr Thr Phe Thr Asp Tyr
               20                  25                  30
   Ala Met His Trp Val Lys Gln Ser Pro Gly Lys Ser Leu Glu Trp Ile
           35                  40                  45
   Gly Val Ile Ser Thr Lys Tyr Gly Lys Thr Asn Tyr Asn Pro Ser Phe
       50                  55                  60
   Gln Gly Gln Ala Thr Met Thr Val Asp Lys Ser Ser Ser Thr Ala Tyr
   65                  70                  75                  80
   Met Glu Leu Ala Ser Leu Lys Ala Ser Asp Ser Ala Ile Tyr Tyr Cys
                   85                  90                  95
   Ala Arg Gly Asp Asp Gly Tyr Ser Trp Gly Gln Gly Thr Ser Val Thr
               100                 105                 110
   Val Ser Ser Ala Ser Thr Lys Gly Pro Ser Val Phe Pro Leu Ala Pro
           115                 120                 125
   Cys Ser Arg Ser Thr Ser Glu Ser Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Lys Thr Tyr Thr Cys Asn Val Asp His Lys Pro Ser Asn Thr Lys
           195                 200                 205
   Val Asp Lys Arg Val Glu Ser Lys Tyr Gly Pro Pro Cys Pro Pro Cys
       210                 215                 220
   Pro Ala Pro Glu Phe Glu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser Gln Glu Asp Pro Glu Val Gln Phe Asn Trp
               260                 265                 270
   Tyr Val Asp Gly Val Glu Val His Asn Ala Lys Thr Lys Pro Arg Glu
           275                 280                 285
   Glu Gln Phe Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Gly Leu Pro Ser Ser Ile Glu Lys Thr Ile Ser Lys Ala Lys Gly
                   325                 330                 335
   Gln Pro Arg Glu Pro Gln Val Tyr Thr Leu Pro Pro Ser Gln Glu Glu
               340                 345                 350
   Met Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Arg Leu Thr Val Asp Lys Ser Arg Trp Gln Glu Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Leu Gly
           435                 440
<210> 3
   <211> 660
   <212> DNA
   <213> Artificial
<220>
   <223> Séquence humanisée
<220>
   <221> CDS
   <222> (1)..(660)
<400> 3
   gag atc gtg atg acc caa act cca ctc tcc ctg cct gtc agt ctt gga       48
   Glu Ile Val Met Thr Gln Thr Pro Leu Ser Leu Pro Val Ser Leu Gly
   1               5                   10                  15
   gat aga gcc tcc atc tct tgc aga tct ggt cag agc ctt gtg cac agt       96
   Asp Arg Ala Ser Ile Ser Cys Arg Ser Gly Gln Ser Leu Val His Ser
               20                  25                  30
   aat gga aac acc tat ctg cat tgg tac ctg cag aag cca ggc cag tct      144
   Asn Gly Asn Thr Tyr Leu His Trp Tyr Leu Gln Lys Pro Gly Gln Ser
           35                  40                  45
   cca aag ctc ctg atc tat aca gtt tcc aac cga ttt tct ggg gtc ccg      192
   Pro Lys Leu Leu Ile Tyr Thr Val Ser Asn Arg Phe Ser Gly Val Pro
       50                  55                  60
   gac agg ttc agt ggc agt gga tca ggg tca gat ttc aca ctc acc atc      240
   Asp Arg Phe Ser Gly Ser Gly Ser Gly Ser Asp Phe Thr Leu Thr Ile
   65                  70                  75                  80
   agc aga gtg gag gct gag gat ctg gga gtt tat ttc tgc tct caa aat      288
   Ser Arg Val Glu Ala Glu Asp Leu Gly Val Tyr Phe Cys Ser Gln Asn
                   85                  90                  95
   aca ttt gtt cct tgg acg ttc ggt gga ggc acc aag ctg gaa atc aaa      336
   Thr Phe Val Pro Trp Thr Phe Gly Gly Gly Thr Lys Leu Glu Ile Lys
               100                 105                 110
   cgt acg gtg gct gca cca tct gtc ttc atc ttc ccg cca tct gat gag      384
   Arg Thr Val Ala Ala Pro Ser Val Phe Ile Phe Pro Pro Ser Asp Glu
           115                 120                 125
   cag ttg aaa tct gga act gcc tct gtt gtg tgc ctg ctg aat aac ttc      432
   Gln Leu Lys Ser Gly Thr Ala Ser Val Val Cys Leu Leu Asn Asn Phe
       130                 135                 140
   tat ccc aga gag gcc aaa gta cag tgg aag gtg gat aac gcc ctc caa      480
   Tyr Pro Arg Glu Ala Lys Val Gln Trp Lys Val Asp Asn Ala Leu Gln
   145                 150                 155                 160
   tcg ggt aac tcc cag gag agt gtc aca gag cag gac agc aag gac agc      528
   Ser Gly Asn Ser Gln Glu Ser Val Thr Glu Gln Asp Ser Lys Asp Ser
                   165                 170                 175
   acc tac agc ctc agc agc acc ctg acg ctg agc aaa gca gac tac gag      576
   Thr Tyr Ser Leu Ser Ser Thr Leu Thr Leu Ser Lys Ala Asp Tyr Glu
               180                 185                 190
   aaa cac aaa gtc tac gcc tgc gaa gtc acc cat cag ggc ctg agc tcg      624
   Lys His Lys Val Tyr Ala Cys Glu Val Thr His Gln Gly Leu Ser Ser
           195                 200                 205
   ccc gtc aca aag agc ttc aac agg gga gag tgt tga                      660
   Pro Val Thr Lys Ser Phe Asn Arg Gly Glu Cys
       210                 215
<210> 4
   <211> 219
   <212> PRT
   <213> Artificial
<220>
   <223> Synthetic Construct
<400> 4
   Glu Ile Val Met Thr Gln Thr Pro Leu Ser Leu Pro Val Ser Leu Gly
   1               5                   10                  15
   Asp Arg Ala Ser Ile Ser Cys Arg Ser Gly Gln Ser Leu Val His Ser
               20                  25                  30
   Asn Gly Asn Thr Tyr Leu His Trp Tyr Leu Gln Lys Pro Gly Gln Ser
           35                  40                  45
   Pro Lys Leu Leu Ile Tyr Thr Val Ser Asn Arg Phe Ser Gly Val Pro
       50                  55                  60
   Asp Arg Phe Ser Gly Ser Gly Ser Gly Ser Asp Phe Thr Leu Thr Ile
   65                  70                  75                  80
   Ser Arg Val Glu Ala Glu Asp Leu Gly Val Tyr Phe Cys Ser Gln Asn
                   85                  90                  95
   Thr Phe Val Pro Trp Thr Phe Gly Gly Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 5
   <211> 345
   <212> DNA
   <213> Artificial
<220>
   <223> Séquence humanisée
<220>
   <221> CDS
   <222> (1)..(345)
<400> 5
   gag gtc cag ctg cag cag tct ggg cct gag gtg gtg aag cct ggg gtc      48
   Glu Val Gln Leu Gln Gln Ser Gly Pro Glu Val Val Lys Pro Gly Val
   1               5                   10                  15
   tca gtg aag att tcc tgc aag ggt tcc ggc tac aca ttc act gat tat      96
   Ser Val Lys Ile Ser Cys Lys Gly Ser Gly Tyr Thr Phe Thr Asp Tyr
               20                  25                  30
   gct atg cac tgg gtg aag cag agt cct ggc aag agt ctg gag tgg att     144
   Ala Met His Trp Val Lys Gln Ser Pro Gly Lys Ser Leu Glu Trp Ile
           35                  40                  45
   gga gtt att agt act aag tat ggt aag aca aac tac aac ccc agc ttt     192
   Gly Val Ile Ser Thr Lys Tyr Gly Lys Thr Asn Tyr Asn Pro Ser Phe
       50                  55                  60
   cag ggc cag gcc aca atg act gtt gac aaa tcc tcc agc aca gcc tat     240
   Gln Gly Gln Ala Thr Met Thr Val Asp Lys Ser Ser Ser Thr Ala Tyr
   65                  70                  75                  80
   atg gag ctt gcc agc ttg aag gcc tcc gat tct gcc atc tat tac tgt     288
   Met Glu Leu Ala Ser Leu Lys Ala Ser Asp Ser Ala Ile Tyr Tyr Cys
                   85                  90                  95
   gca aga ggg gac gat ggt tat tcc tgg ggt caa gga acc tca gtc acc     336
   Ala Arg Gly Asp Asp Gly Tyr Ser Trp Gly Gln Gly Thr Ser Val Thr
               100                 105                 110
   gtc tcc agc                                                    345
   Val Ser Ser
           115
<210> 6
   <211> 115
   <212> PRT
   <213> Artificial
<220>
   <223> Synthetic Construct
<400> 6
   Glu Val Gln Leu Gln Gln Ser Gly Pro Glu Val Val Lys Pro Gly Val
   1               5                   10                  15
   Ser Val Lys Ile Ser Cys Lys Gly Ser Gly Tyr Thr Phe Thr Asp Tyr
               20                  25                  30
   Ala Met His Trp Val Lys Gln Ser Pro Gly Lys Ser Leu Glu Trp Ile
           35                  40                  45
   Gly Val Ile Ser Thr Lys Tyr Gly Lys Thr Asn Tyr Asn Pro Ser Phe
       50                  55                  60
   Gln Gly Gln Ala Thr Met Thr Val Asp Lys Ser Ser Ser Thr Ala Tyr
   65                  70                  75                  80
   Met Glu Leu Ala Ser Leu Lys Ala Ser Asp Ser Ala Ile Tyr Tyr Cys
                   85                  90                  95
   Ala Arg Gly Asp Asp Gly Tyr Ser Trp Gly Gln Gly Thr Ser Val Thr
               100                 105                 110
   Val Ser Ser
           115
<210> 7
   <211> 339
   <212> DNA
   <213> Artificial
<220>
   <223> Séquence humanisée
<220>
   <221> CDS
   <222> (1)..(339)
<400> 7
   gag atc gtg atg acc caa act cca ctc tcc ctg cct gtc agt ctt gga        48
   Glu Ile Val Met Thr Gln Thr Pro Leu Ser Leu Pro Val Ser Leu Gly
   1               5                   10                  15
   gat aga gcc tcc atc tct tgc aga tct ggt cag agc ctt gtg cac agt        96
   Asp Arg Ala Ser Ile Ser Cys Arg Ser Gly Gln Ser Leu Val His Ser
               20                  25                  30
   aat gga aac acc tat ctg cat tgg tac ctg cag aag cca ggc cag tct       144
   Asn Gly Asn Thr Tyr Leu His Trp Tyr Leu Gln Lys Pro Gly Gln Ser
           35                  40                  45
   cca aag ctc ctg atc tat aca gtt tcc aac cga ttt tct ggg gtc ccg       192
   Pro Lys Leu Leu Ile Tyr Thr Val Ser Asn Arg Phe Ser Gly Val Pro
       50                  55                  60
   gac agg ttc agt ggc agt gga tca ggg tca gat ttc aca ctc acc atc       240
   Asp Arg Phe Ser Gly Ser Gly Ser Gly Ser Asp Phe Thr Leu Thr Ile
   65                  70                  75                  80
   agc aga gtg gag gct gag gat ctg gga gtt tat ttc tgc tct caa aat       288
   Ser Arg Val Glu Ala Glu Asp Leu Gly Val Tyr Phe Cys Ser Gln Asn
                   85                  90                  95
   aca ttt gtt cct tgg acg ttc ggt gga ggc acc aag ctg gaa atc aaa       336
   Thr Phe Val Pro Trp Thr Phe Gly Gly Gly Thr Lys Leu Glu Ile Lys
               100                 105                 110
   cgt                                                                   339
   Arg
<210> 8
   <211> 113
   <212> PRT
   <213> Artificial
<220>
   <223> Synthetic Construct
<400> 8
   Glu Ile Val Met Thr Gln Thr Pro Leu Ser Leu Pro Val Ser Leu Gly
   1               5                   10                  15
   Asp Arg Ala Ser Ile Ser Cys Arg Ser Gly Gln Ser Leu Val His Ser
               20                  25                  30
   Asn Gly Asn Thr Tyr Leu His Trp Tyr Leu Gln Lys Pro Gly Gln Ser
           35                  40                  45
   Pro Lys Leu Leu Ile Tyr Thr Val Ser Asn Arg Phe Ser Gly Val Pro
       50                  55                  60
   Asp Arg Phe Ser Gly Ser Gly Ser Gly Ser Asp Phe Thr Leu Thr Ile
   65                  70                  75                  80
   Ser Arg Val Glu Ala Glu Asp Leu Gly Val Tyr Phe Cys Ser Gln Asn
                   85                  90                  95
   Thr Phe Val Pro Trp Thr Phe Gly Gly Gly Thr Lys Leu Glu Ile Lys
               100                 105                 110
   Arg
<210> 9
   <211> 33
   <212> DNA
   <213> Mus sp.
<220>
   <221> CDS
   <222> (1)..(33)
<400> 9
   tcc ggc tac aca ttc act gat tat gct atg cac 33
   Ser Gly Tyr Thr Phe Thr Asp Tyr Ala Met His
   1               5                   10
<210> 10
   <211> 11
   <212> PRT
   <213> Mus sp.
<400> 10
   Ser Gly Tyr Thr Phe Thr Asp Tyr Ala Met His
   1               5                   10
<210> 11
   <211> 30
   <212> DNA
   <213> Mus sp.
<220>
   <221> CDS
   <222> (1)..(30)
<400> 11
   gtt att agt act aag tat ggt aag aca aac                                30
   Val Ile Ser Thr Lys Tyr Gly Lys Thr Asn
   1               5                   10
<210> 12
   <211> 10
   <212> PRT
   <213> Mus sp.
<400> 12
   Val Ile Ser Thr Lys Tyr Gly Lys Thr Asn
   1               5                   10
<210> 13
   <211> 18
   <212> DNA
   <213> Mus sp.
<220>
   <221> CDS
   <222> (1)..(18)
<400> 13
   ggg gac gat ggt tat tcc                                                18
   Gly Asp Asp Gly Tyr Ser
   1               5
<210> 14
   <211> 6
   <212> PRT
   <213> Mus sp.
<400> 14
   Gly Asp Asp Gly Tyr Ser
   1               5
<210> 15
   <211> 48
   <212> DNA
   <213> Mus sp.
<220>
   <221> CDS
   <222> (1)..(48)
<400> 15
   aga tct ggt cag agc ctt gtg cac agt aat gga aac acc tat ctg cat        48
   Arg Ser Gly Gln Ser Leu Val His Ser Asn Gly Asn Thr Tyr Leu His
   1               5                   10                  15
<210> 16
   <211> 16
   <212> PRT
   <213> Mus sp.
<400> 16
   Arg Ser Gly Gln Ser Leu Val His Ser Asn Gly Asn Thr Tyr Leu His
   1               5                   10                  15
<210> 17
   <211> 24
   <212> DNA
   <213> Mus sp.
<220>
   <221> CDS
   <222> (1)..(24)
<400> 17
   aca gtt tcc aac cga ttt tct ggg                                        24
   Thr Val Ser Asn Arg Phe Ser Gly
   1               5
<210> 18
   <211> 8
   <212> PRT
   <213> Mus sp.
<400> 18
   Thr Val Ser Asn Arg Phe Ser Gly
   1               5
<210> 19
   <211> 27
   <212> DNA
   <213> Mus sp.
<220>
   <221> CDS
   <222> (1)..(27)
<400> 19
   tct caa aat aca ttt gtt cct tgg acg                                    27
   Ser Gln Asn Thr Phe Val Pro Trp Thr
   1               5
<210> 20
   <211> 9
   <212> PRT
   <213> Mus sp.
<400> 20
   Ser Gln Asn Thr Phe Val Pro Trp Thr
   1               5
<210> 21
   <211> 660
   <212> DNA
   <213> Artificial
<220>
   <223> Séquence humanisée
<220>
   <221> CDS
   <222> (1)..(660)
<400> 21
   gag atc gtg atg acc caa act cca ctc tcc ctg cct gtc agt ctt gga         48
   Glu Ile Val Met Thr Gln Thr Pro Leu Ser Leu Pro Val Ser Leu Gly
   1               5                   10                  15
   gat aga gcc tcc atc tct tgc aga tct ggt cag agc ctt gtg cac agt        96
   Asp Arg Ala Ser Ile Ser Cys Arg Ser Gly Gln Ser Leu Val His Ser
               20                  25                  30
   aat acc aac acc tat ctg cat tgg tac ctg cag aag cca ggc cag tct       144
   Asn Thr Asn Thr Tyr Leu His Trp Tyr Leu Gln Lys Pro Gly Gln Ser
           35                  40                  45
   cca aag ctc ctg atc tat aca gtt tcc aac cga ttt tct ggg gtc ccg       192
   Pro Lys Leu Leu Ile Tyr Thr Val Ser Asn Arg Phe Ser Gly Val Pro
       50                  55                  60
   gac agg ttc agt ggc agt gga tca ggg tca gat ttc aca ctc acc atc       240
   Asp Arg Phe Ser Gly Ser Gly Ser Gly Ser Asp Phe Thr Leu Thr Ile
   65                  70                  75                  80
   agc aga gtg gag gct gag gat ctg gga gtt tat ttc tgc tct caa aat       288
   Ser Arg Val Glu Ala Glu Asp Leu Gly Val Tyr Phe Cys Ser Gln Asn
                   85                  90                  95
   aca ttt gtt cct tgg acg ttc ggt gga ggc acc aag ctg gaa atc aaa       336
   Thr Phe Val Pro Trp Thr Phe Gly Gly Gly Thr Lys Leu Glu Ile Lys
               100                 105                 110
   cgt acg gtg gct gca cca tct gtc ttc atc ttc ccg cca tct gat gag       384
   Arg Thr Val Ala Ala Pro Ser Val Phe Ile Phe Pro Pro Ser Asp Glu
           115                 120                 125
   cag ttg aaa tct gga act gcc tct gtt gtg tgc ctg ctg aat aac ttc       432
   Gln Leu Lys Ser Gly Thr Ala Ser Val Val Cys Leu Leu Asn Asn Phe
       130                 135                 140
   tat ccc aga gag gcc aaa gta cag tgg aag gtg gat aac gcc ctc caa       480
   Tyr Pro Arg Glu Ala Lys Val Gln Trp Lys Val Asp Asn Ala Leu Gln
   145                 150                 155                 160
   tcg ggt aac tcc cag gag agt gtc aca gag cag gac agc aag gac agc       528
   Ser Gly Asn Ser Gln Glu Ser Val Thr Glu Gln Asp Ser Lys Asp Ser
                   165                 170                 175
   acc tac agc ctc agc agc acc ctg acg ctg agc aaa gca gac tac gag       576
   Thr Tyr Ser Leu Ser Ser Thr Leu Thr Leu Ser Lys Ala Asp Tyr Glu
               180                 185                 190
   aaa cac aaa gtc tac gcc tgc gaa gtc acc cat cag ggc ctg agc tcg       624
   Lys His Lys Val Tyr Ala Cys Glu Val Thr His Gln Gly Leu Ser Ser
           195                 200                 205
   ccc gtc aca aag agc ttc aac agg gga gag tgt tga                       660
   Pro Val Thr Lys Ser Phe Asn Arg Gly Glu Cys
       210                 215
<210> 22
   <211> 219
   <212> PRT
   <213> Artificial
<220>
   <223> Synthetic Construct
<400> 22
   Glu Ile Val Met Thr Gln Thr Pro Leu Ser Leu Pro Val Ser Leu Gly
   1               5                   10                  15
   Asp Arg Ala Ser Ile Ser Cys Arg Ser Gly Gln Ser Leu Val His Ser
               20                  25                  30
   Asn Thr Asn Thr Tyr Leu His Trp Tyr Leu Gln Lys Pro Gly Gln Ser
           35                  40                  45
   Pro Lys Leu Leu Ile Tyr Thr Val Ser Asn Arg Phe Ser Gly Val Pro
       50                  55                  60
   Asp Arg Phe Ser Gly Ser Gly Ser Gly Ser Asp Phe Thr Leu Thr Ile
   65                  70                  75                  80
   Ser Arg Val Glu Ala Glu Asp Leu Gly Val Tyr Phe Cys Ser Gln Asn
                   85                  90                  95
   Thr Phe Val Pro Trp Thr Phe Gly Gly Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 23
   <211> 339
   <212> DNA
   <213> Artificial
<220>
   <223> Séquence humanisée
<220>
   <221> CDS
   <222> (1)..(339)
<400> 23
   gag atc gtg atg acc caa act cca ctc tcc ctg cct gtc agt ctt gga        48
   Glu Ile Val Met Thr Gln Thr Pro Leu Ser Leu Pro Val Ser Leu Gly
   1               5                   10                  15
   gat aga gcc tcc atc tct tgc aga tct ggt cag agc ctt gtg cac agt        96
   Asp Arg Ala Ser Ile Ser Cys Arg Ser Gly Gln Ser Leu Val His Ser
               20                  25                  30
   aat acc aac acc tat ctg cat tgg tac ctg cag aag cca ggc cag tct       144
   Asn Thr Asn Thr Tyr Leu His Trp Tyr Leu Gln Lys Pro Gly Gln Ser
           35                  40                  45
   cca aag ctc ctg atc tat aca gtt tcc aac cga ttt tct ggg gtc ccg       192
   Pro Lys Leu Leu Ile Tyr Thr Val Ser Asn Arg Phe Ser Gly Val Pro
       50                  55                  60
   gac agg ttc agt ggc agt gga tca ggg tca gat ttc aca ctc acc atc       240
   Asp Arg Phe Ser Gly Ser Gly Ser Gly Ser Asp Phe Thr Leu Thr Ile
   65                  70                  75                  80
   agc aga gtg gag gct gag gat ctg gga gtt tat ttc tgc tct caa aat       288
   Ser Arg Val Glu Ala Glu Asp Leu Gly Val Tyr Phe Cys Ser Gln Asn
                   85                  90                  95
   aca ttt gtt cct tgg acg ttc ggt gga ggc acc aag ctg gaa atc aaa       336
   Thr Phe Val Pro Trp Thr Phe Gly Gly Gly Thr Lys Leu Glu Ile Lys
               100                 105                 110
   cgt                                                                   339
   Arg
<210> 24
   <211> 113
   <212> PRT
   <213> Artificial
<220>
   <223> Synthetic Construct
<400> 24
   Glu Ile Val Met Thr Gln Thr Pro Leu Ser Leu Pro Val Ser Leu Gly
   1               5                   10                  15
   Asp Arg Ala Ser Ile Ser Cys Arg Ser Gly Gln Ser Leu Val His Ser
               20                  25                  30
   Asn Thr Asn Thr Tyr Leu His Trp Tyr Leu Gln Lys Pro Gly Gln Ser
           35                  40                  45
   Pro Lys Leu Leu Ile Tyr Thr Val Ser Asn Arg Phe Ser Gly Val Pro
       50                  55                  60
   Asp Arg Phe Ser Gly Ser Gly Ser Gly Ser Asp Phe Thr Leu Thr Ile
   65                  70                  75                  80
   Ser Arg Val Glu Ala Glu Asp Leu Gly Val Tyr Phe Cys Ser Gln Asn
                   85                  90                  95
   Thr Phe Val Pro Trp Thr Phe Gly Gly Gly Thr Lys Leu Glu Ile Lys
               100                 105                 110
   Arg
<210> 25
   <211> 1326
   <212> DNA
   <213> Artificial
<220>
   <223> Séquence humanisée
<220>
   <221> CDS
   <222> (1)..(1326)
<400> 25
   gag gtc cag ctg cag cag tct ggg cct gag gtg gtg aag cct ggg gtc      48
   Glu Val Gln Leu Gln Gln Ser Gly Pro Glu Val Val Lys Pro Gly Val
   1               5                   10                  15
   tca gtg aag att tcc tgc aag ggt tcc ggc tac aca ttc act gat tat      96
   Ser Val Lys Ile Ser Cys Lys Gly Ser Gly Tyr Thr Phe Thr Asp Tyr
               20                  25                  30
   gct atg cac tgg gtg aag cag agt cct ggc aag agt ctg gag tgg att     144
   Ala Met His Trp Val Lys Gln Ser Pro Gly Lys Ser Leu Glu Trp Ile
           35                  40                  45
   gga gtt att agt act aag tat ggt aag aca aac tac aac ccc agc ttt     192
   Gly Val Ile Ser Thr Lys Tyr Gly Lys Thr Asn Tyr Asn Pro Ser Phe
       50                  55                  60
   cag ggc cag gcc aca atg act gtt gac aaa tcc tcc agc aca gcc tat     240
   Gln Gly Gln Ala Thr Met Thr Val Asp Lys Ser Ser Ser Thr Ala Tyr
   65                  70                  75                  80
   atg gag ctt gcc agc ttg aag gcc tcc gat tct gcc atc tat tac tgt     288
   Met Glu Leu Ala Ser Leu Lys Ala Ser Asp Ser Ala Ile Tyr Tyr Cys
                   85                  90                  95
   gca aga ggg gac gag ggt tat tcc tgg ggt caa gga acc tca gtc acc     336
   Ala Arg Gly Asp Glu Gly Tyr Ser Trp Gly Gln Gly Thr Ser Val Thr
               100                 105                 110
   gtc tcc agc gct tct acc aag ggc cct tcc gtg ttc cct ctg gcc cct     384
   Val Ser Ser Ala Ser Thr Lys Gly Pro Ser Val Phe Pro Leu Ala Pro
           115                 120                 125
   tgc tcc cgg tcc acc tcc gag tcc acc gcc gct ctg ggc tgc ctg gtg       432
   Cys Ser Arg Ser Thr Ser Glu Ser Thr Ala Ala Leu Gly Cys Leu Val
       130                 135                 140
   aag gac tac ttc cct gag cct gtg acc gtg tcc tgg aac tct ggc gcc       480
   Lys Asp Tyr Phe Pro Glu Pro Val Thr Val Ser Trp Asn Ser Gly Ala
   145                 150                 155                 160
   ctg acc tcc ggc gtg cac acc ttc cct gcc gtg ctg cag tcc tcc ggc       528
   Leu Thr Ser Gly Val His Thr Phe Pro Ala Val Leu Gln Ser Ser Gly
                   165                 170                 175
   ctg tac tcc ctg tcc tcc gtg gtg acc gtg cct tcc tcc tcc ctg ggc       576
   Leu Tyr Ser Leu Ser Ser Val Val Thr Val Pro Ser Ser Ser Leu Gly
               180                 185                 190
   acc aag acc tac acc tgt aac gtg gac cac aag cct tcc aac acc aag       624
   Thr Lys Thr Tyr Thr Cys Asn Val Asp His Lys Pro Ser Asn Thr Lys
           195                 200                 205
   gtg gac aag cgg gtg gag tcc aag tac ggc cct cct tgc cct ccc tgc       672
   Val Asp Lys Arg Val Glu Ser Lys Tyr Gly Pro Pro Cys Pro Pro Cys
       210                 215                 220
   cct gcc cct gag ttc gag ggc gga cct agc gtg ttc ctg ttc cct cct       720
   Pro Ala Pro Glu Phe Glu Gly Gly Pro Ser Val Phe Leu Phe Pro Pro
   225                 230                 235                 240
   aag cct aag gac acc ctg atg atc tcc cgg acc cct gag gtg acc tgt       768
   Lys Pro Lys Asp Thr Leu Met Ile Ser Arg Thr Pro Glu Val Thr Cys
                   245                 250                 255
   gtg gtg gtg gac gtg tcc cag gag gac cct gag gtc cag ttc aac tgg       816
   Val Val Val Asp Val Ser Gln Glu Asp Pro Glu Val Gln Phe Asn Trp
               260                 265                 270
   tac gtg gac ggc gtg gag gtg cac aac gcc aag acc aag cct cgg gag       864
   Tyr Val Asp Gly Val Glu Val His Asn Ala Lys Thr Lys Pro Arg Glu
           275                 280                 285
   gag cag ttc aat tcc acc tac cgg gtg gtg tct gtg ctg acc gtg ctg       912
   Glu Gln Phe Asn Ser Thr Tyr Arg Val Val Ser Val Leu Thr Val Leu
       290                 295                 300
   cac cag gac tgg ctg aac ggc aaa gaa tac aag tgt aag gtc tcc aac       960
   His Gln Asp Trp Leu Asn Gly Lys Glu Tyr Lys Cys Lys Val Ser Asn
   305                 310                 315                 320
   aag ggc ctg ccc tcc tcc atc gag aaa acc atc tcc aag gcc aag ggc      1008
   Lys Gly Leu Pro Ser Ser Ile Glu Lys Thr Ile Ser Lys Ala Lys Gly
                   325                 330                 335
   cag cct agg gag cct cag gtg tac acc ctg cct cct agc cag gaa gag      1056
   Gln Pro Arg Glu Pro Gln Val Tyr Thr Leu Pro Pro Ser Gln Glu Glu
               340                 345                 350
   atg acc aag aac cag gtg tcc ctg acc tgt ctg gtg aag ggc ttc tac      1104
   Met Thr Lys Asn Gln Val Ser Leu Thr Cys Leu Val Lys Gly Phe Tyr
           355                 360                 365
   cct tcc gac atc gcc gtg gag tgg gag tcc aac ggc cag cct gag aac     1152
   Pro Ser Asp Ile Ala Val Glu Trp Glu Ser Asn Gly Gln Pro Glu Asn
       370                 375                 380
   aac tac aag acc acc cct cct gtg ctg gac tcc gac ggc tcc ttc ttc     1200
   Asn Tyr Lys Thr Thr Pro Pro Val Leu Asp Ser Asp Gly Ser Phe Phe
   385                 390                 395                 400
   ctg tac tcc agg ctg acc gtg gac aag tcc cgg tgg cag gag ggc aac     1248
   Leu Tyr Ser Arg Leu Thr Val Asp Lys Ser Arg Trp Gln Glu Gly Asn
                   405                 410                 415
   gtc ttt tcc tgc tcc gtg atg cac gag gcc ctg cac aac cac tac acc     1296
   Val Phe Ser Cys Ser Val Met His Glu Ala Leu His Asn His Tyr Thr
               420                 425                 430
   cag aag tcc ctg tcc ctg tct ctg ggc tga                             1326
   Gln Lys Ser Leu Ser Leu Ser Leu Gly
           435                 440
<210> 26
   <211> 441
   <212> PRT
   <213> Artificial
<220>
   <223> Synthetic Construct
<400> 26
   Glu Val Gln Leu Gln Gln Ser Gly Pro Glu Val Val Lys Pro Gly Val
   1               5                   10                  15
   Ser Val Lys Ile Ser Cys Lys Gly Ser Gly Tyr Thr Phe Thr Asp Tyr
               20                  25                  30
   Ala Met His Trp Val Lys Gln Ser Pro Gly Lys Ser Leu Glu Trp Ile
           35                  40                  45
   Gly Val Ile Ser Thr Lys Tyr Gly Lys Thr Asn Tyr Asn Pro Ser Phe
       50                  55                  60
   Gln Gly Gln Ala Thr Met Thr Val Asp Lys Ser Ser Ser Thr Ala Tyr
   65                  70                  75                  80
   Met Glu Leu Ala Ser Leu Lys Ala Ser Asp Ser Ala Ile Tyr Tyr Cys
                   85                  90                  95
   Ala Arg Gly Asp Glu Gly Tyr Ser Trp Gly Gln Gly Thr Ser Val Thr
               100                 105                 110
   Val Ser Ser Ala Ser Thr Lys Gly Pro Ser Val Phe Pro Leu Ala Pro
           115                 120                 125
   Cys Ser Arg Ser Thr Ser Glu Ser Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Lys Thr Tyr Thr Cys Asn Val Asp His Lys Pro Ser Asn Thr Lys
           195                 200                 205
   Val Asp Lys Arg Val Glu Ser Lys Tyr Gly Pro Pro Cys Pro Pro Cys
       210                 215                 220
   Pro Ala Pro Glu Phe Glu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser Gln Glu Asp Pro Glu Val Gln Phe Asn Trp
               260                 265                 270
   Tyr Val Asp Gly Val Glu Val His Asn Ala Lys Thr Lys Pro Arg Glu
           275                 280                 285
   Glu Gln Phe Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Gly Leu Pro Ser Ser Ile Glu Lys Thr Ile Ser Lys Ala Lys Gly
                   325                 330                 335
   Gln Pro Arg Glu Pro Gln Val Tyr Thr Leu Pro Pro Ser Gln Glu Glu
               340                 345                 350
   Met Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Arg Leu Thr Val Asp Lys Ser Arg Trp Gln Glu Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Leu Gly
           435                 440
<210> 27
   <211> 345
   <212> DNA
   <213> Artificial
<220>
   <223> Séquence humanisée
<220>
   <221> CDS
   <222> (1)..(345)
<400> 27
   gag gtc cag ctg cag cag tct ggg cct gag gtg gtg aag cct ggg gtc       48
   Glu Val Gln Leu Gln Gln Ser Gly Pro Glu Val Val Lys Pro Gly Val
   1               5                   10                  15
   tca gtg aag att tcc tgc aag ggt tcc ggc tac aca ttc act gat tat       96
   Ser Val Lys Ile Ser Cys Lys Gly Ser Gly Tyr Thr Phe Thr Asp Tyr
               20                  25                  30
   gct atg cac tgg gtg aag cag agt cct ggc aag agt ctg gag tgg att      144
   Ala Met His Trp Val Lys Gln Ser Pro Gly Lys Ser Leu Glu Trp Ile
           35                  40                  45
   gga gtt att agt act aag tat ggt aag aca aac tac aac ccc agc ttt      192
   Gly Val Ile Ser Thr Lys Tyr Gly Lys Thr Asn Tyr Asn Pro Ser Phe
       50                  55                  60
   cag ggc cag gcc aca atg act gtt gac aaa tcc tcc agc aca gcc tat      240
   Gln Gly Gln Ala Thr Met Thr Val Asp Lys Ser Ser Ser Thr Ala Tyr
   65                  70                  75                  80
   atg gag ctt gcc agc ttg aag gcc tcc gat tct gcc atc tat tac tgt      288
   Met Glu Leu Ala Ser Leu Lys Ala Ser Asp Ser Ala Ile Tyr Tyr Cys
                   85                  90                  95
   gca aga ggg gac gag ggt tat tcc tgg ggt caa gga acc tca gtc acc      336
   Ala Arg Gly Asp Glu Gly Tyr Ser Trp Gly Gln Gly Thr Ser Val Thr
               100                 105                 110
   gtc tcc agc                                                          345
   Val Ser Ser
           115
<210> 28
   <211> 115
   <212> PRT
   <213> Artificial
<220>
   <223> Synthetic Construct
<400> 28
   Glu Val Gln Leu Gln Gln Ser Gly Pro Glu Val Val Lys Pro Gly Val
   1               5                   10                  15
   Ser Val Lys Ile Ser Cys Lys Gly Ser Gly Tyr Thr Phe Thr Asp Tyr
               20                  25                  30
   Ala Met His Trp Val Lys Gln Ser Pro Gly Lys Ser Leu Glu Trp Ile
           35                  40                  45
   Gly Val Ile Ser Thr Lys Tyr Gly Lys Thr Asn Tyr Asn Pro Ser Phe
       50                  55                  60
   Gln Gly Gln Ala Thr Met Thr Val Asp Lys Ser Ser Ser Thr Ala Tyr
   65                  70                  75                  80
   Met Glu Leu Ala Ser Leu Lys Ala Ser Asp Ser Ala Ile Tyr Tyr Cys
                   85                  90                  95
   Ala Arg Gly Asp Glu Gly Tyr Ser Trp Gly Gln Gly Thr Ser Val Thr
               100                 105                 110
   Val Ser Ser
           115
<210> 29
   <211> 18
   <212> DNA
   <213> Mus sp.
<220>
   <221> CDS
   <222> (1)..(18)
<400> 29
   ggg gac gag ggt tat tcc                                                  18
   Gly Asp Glu Gly Tyr Ser
   1               5
<210> 30
   <211> 6
   <212> PRT
   <213> Mus sp.
<400> 30
   Gly Asp Glu Gly Tyr Ser
   1               5
<210> 31
   <211> 48
   <212> DNA
   <213> Mus sp.
<220>
   <221> CDS
   <222> (1)..(48)
<400> 31
   aga tct ggt cag agc ctt gtg cac agt aat acc aac acc tat ctg cat      48
   Arg Ser Gly Gln Ser Leu Val His Ser Asn Thr Asn Thr Tyr Leu His
   1               5                   10                  15
<210> 32
   <211> 16
   <212> PRT
   <213> Mus sp.
<400> 32
   Arg Ser Gly Gln Ser Leu Val His Ser Asn Thr Asn Thr Tyr Leu His
   1               5                   10                  15